# EUROPEAN PATENT SPECIFICATION

(11) **EP 2 291 382 B1**
(45) Date of publication and mention of the grant of the patent: **14.12.2011**
(21) Application number: 09749727.5
(22) Date of filing: 11.05.2009
(51) Int. Cl.: C07D 491/10

(54) **TIME TEMPERATURE INDICATOR COMPRISING INDOLENIN BASED SPIROPYRANS CONTAINING A N-ACETYLAMIDO OR N- ACETYLESTER SIDE CHAIN**
ZEIT-TEMPERATUR-ANZEIGE MIT SPIROPYRANEN AUF INDOLENINBASIS MIT EINER N-ACETYLAMID- ODER N- ACETYLESTERSEITENKETTE
INDICATEUR TEMPS-TEMPÉRATURE COMPRENANT DES SPIROPYRANES À BASE D'INDOLÉNINE CONTENANT UNE CHAÎNE LATÉRALE N-ACÉTYLAMIDO OU N-ACÉTYLESTER

(30) Priority: 21.05.2008 EP 08156605
(43) Date of publication of application: 09.03.2011
(73) Proprietor: BASF SE, 67056 Ludwigshafen (DE)
(72) Inventor: FEILER, Leonhard, 79589 Binzen (DE); RAIMANN, Thomas, CH-4334 Sisseln (CH)
(86) International application number: PCT/EP2009/055641
(87) International publication number: WO 2009/141237

(56) References cited:
- WO-A-99/39197
- WO-A-2005/075978
- DE-A1- 19 646 820
- JP-A- 3 261 787
- JP-A- 62 242 686
- US-A- 5 403 702
- M. A. GALBERTSHTAM, N. M. PRZHIYAGALGOVSKAYA, O. R. KHROLOVA, I. B. LAZARENKO, G. K. BOBYLEVA, N. N. SUVOROV: "Photochromic properties of some N-substituted 3,3-dimethyl-6'-nitro-indoline-2-spiro-2'- 2H-chromenes" CHEMISTRY OF HETEROCYCLIC COMPOUNDS, vol. 13, 1977, pages 1309-1313, XP002496051
- NERINGA KLEIZIENE, VIRGINE AMANKAVICIENE, ULF BERG, CARSTEN SCHICKTANZ, KLAUS SCHLOTHAUER, ALGIRDAS SACKUS: "Cyclization of nitrospirobenzopyrans to bridged benzoxazepino[3,2-a]indoles" MONATSHEFTE FÜR CHEMIE, vol. 137, 2006, pages 1109-1117, XP002496052
- I. B. LAZARENKO, N. M. PRZHIYALGOVSKAYA, M. A. GALBERSHTAM, G. K. BOBYLEVA, N. N. SUVOROV: "Synthesis and photochromic properties of indolinospirochromenes with benzyl, ethyl, and acetonyl groups attached to the nitrogen atom" CHEMISTRY OF HETEROCYCLIC COMPOUNDS, vol. 18, no. 10, 1982, pages 1054-1057, XP002496053
- Y. GALAGAN, W.-F. SU: "Fadable ink for time-temperature control of food freshness: novel new time-temperature indicator" FOOD RESEARCH INTERNATIONAL, vol. 41, 2008, pages 653-657, XP022819232

## Description

The present invention relates to time-temperature indicator (TTI) systems comprising indolenin based spiropyrans containing a N-acetylamido or N-acetylester side chain.

Temperature abuse is one of the most frequently observed causes for predated goods spoilage. It is therefore important and desired to monitor the time-temperature history of such perishable goods, preferably, using inexpensive and consumer friendly means. Time temperature indicators are substances that are capable of visually reporting on the summary of the time temperature history of the substance, and consequently, of the perishable good it is associated with. Designed for the end user, time temperature indicators are usually designed to report a clear and visual Yes/No signal.

WO 99/39197 describes the use of photochromic dyes, based on a transfer reaction and embedded in the crystalline state, as active materials for TTIs.

The Japanese Publication JP62242686 (1987) discloses N-substituted spiropyrans having a N-acetylamido side chain -CH₂-CON(alkyl)₂ or -CH₂-CONH₂ or-CH2-CONH(alkyl).

M.A. Galbertshtam describes in Chemistry of heterocyclic compounds, Vol 13, 1977, pages 1309-1313 the photochromic properties of some N-substituted spiropyrans having a N-acetylester side chain. Specifically disclosed are carb-ethoxymethyl side chains -CH₂-COOEt.

WO 2005/075978 describes TTIs based on photochromic indicator compounds. The photochromic reactions of the TTIs taught in WO 2005/075978 are valence isomerization reactions without migration of an atom or chemical group attached to the indicator compound in a time and temperature dependent manner. Preferred indicator compounds include diaryl ethenes and spiroaromatics. The spiroaromatic compounds used in WO 2005/075978 do not have an acetyl amino side chain.

TTIs based on a photochromic indicator compound should, ideally, not be affected by surrounding light. Although there is a large selection of suitable filter systems, there is still a need for photochromic indicators which are improved in terms of photostability because existing filters cannot ensure complete protection against photobleaching and/or photo-degration of the indicator compound.

The problem underlying the present invention is therefore to provide a time-temperature indicator system having an **increased photostability** and which can furthermore allow the monitoring of the temperature of more and of less perishable products.

A novel time-temperature indicator (TTI) system that is based on indolenin based spiropyrans containing a N-acetylamido or N-acetylester side chain as active material solves the above referenced problem.

The present invention therefore relates to a time temperature indicator for indicating a temperature change over time, comprising at least one spiropyran indicator of formula (I) 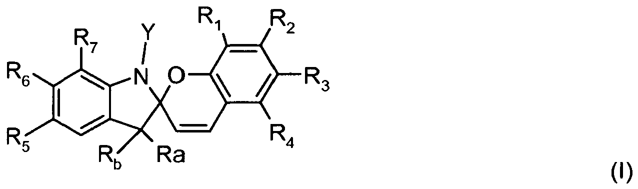 wherein
- R₁: is hydrogen, -C₁-C₁₈ alkoxy, -C₁-C₁₈ alkylthio, -C₁-C₁₈ alkyl-SO-, -C₁-C₁₈ alkyl-SO₂-, phenylthio, phenyl, halogen, -C₁-C₁₈ alkyl or -NO₂;
- R₂: is hydrogen or -C₁-C₁₈ alkoxy;
- R₃: is NO₂ or halogen;
- R₄: is hydrogen, -C₁-C₁₈ alkoxy or halogen;
- R₅: is hydrogen, halogen, -C₁-C₁₈ alkoxy, -COOH, -COO-C₁-C₁₈alkyl, -CF₃ or phenyl;
- R₆: is hydrogen or R₆ and R₇ form together a phenyl ring;
- R₇: is hydrogen;
- Rₐ: is hydrogen or -C₁-C₆ alkyl;
- R_{b}: is hydrogen or -C₁-C₆ alkyl, or together with Rₐ form a 5-6 membered ring;
- Y: is -CH₂-COO-R₈ or -CH₂-CO-N(R₁₀)-R₉; or -CH₂-CO-N(R₁₀)-L-N(R₁₀) CO-CH₂- or Y is -CH₂-CO-O-L'-O-CO-CH₂-
wherein
R₈ is hydrogen, C₃-C₁₈alkyl or R₈ is ethyl with the proviso that R₆ and R₇ form together a phenyl ring;
R₉ is phenyl, mesityl, naphthyl, or higher annelated aromatic systems, phenyl-O-phenyl, phenyl-S-phenyl, or phenyl, naphthyl or the higher annelated aromatic system is once or more than once substituted by halogen, -CF₃, C₁-C₆alkyl, -C₁-C₆ alkoxy, carboxy,-COO-C₁-C₆alkyl , CN, NO₂, N(R₁₁)₂, S-R₁₁ (SO-R₁₁, SO₂-R₁₁), CO-R₁₁, CO-N(R₁₁)₂ (R11=C1-C18alkyl, aryl, substituted aryl) whereby in case of a more than once substitution, the substituent can be the same or different;
R₁₀ is hydrogen, C₁-C₁₈alkyl;
L is 1,3 phenylene or 1,4 phenylene wherein the phenylene linker is optionally substituted by once or more than once by halogen, -CF₃, C₁-C₁₈alkyl, -C₁-C₁₈ alkoxy, carboxy , -COO-C₁-C₁₈alkyl, -CONH₂, -CON(C₁-C₁₈alkyl)₂, nitro; or L is naphthalene, biphenylene or phenylene-O-phenylene wherein the naphthalene, biphenylene or phenylene-O-phenylene linker is optionally substituted once or more than once by halogen, -CF₃, C₁-C₁₈alkyl, -C₁-C₁₈ alkoxy, carboxy , -COO-C₁-C₁₈alkyl, -CONH₂,-CON(C₁-C₁₈alkyl)₂, nitro;
L' is 1,3 phenylene or 1,4 phenylene wherein the phenylene linker is optionally substituted by once or more than once by halogen, -CF₃, C₁-C₁₈alkyl, -C₁-C₁₈ alkoxy, carboxy , -COO-C₁-C₁₈alkyl, -CONH₂, -CON(C₁-C₁₈alkyl)₂, nitro; or L is naphthalene, biphenylene or phenylene-O-phenylene wherein the naphthalene, biphenylene or phenylene-O-phenylene linker is optionally substituted once or more than once by halogen, -CF₃, C₁-C₁₈alkyl, -C₁-C₁₈ alkoxy, carboxy , -COO-C₁-C₁₈alkyl, -CONH₂,-CON(C₁-C₁₈alkyl)₂, nitro.

The proviso for R₈ = ethyl is necessary because of the disclosure of M.A. Galbertshtam describing in Chemistry of heterocyclic compounds, Vol 13, 1977, pages 1309-1313 the photochromic properties of some N-substitued spiropyrans having a N-acetylester side chain. Specifically disclosed are carbethoxymethyl side chains -CH₂-COOEt.

The term "alkyl" refers to linear or branched alkyl groups.

### Preferences:

In one embodiment R₉ is phenyl, mesityl, phenyl-O-phenyl, phenyl-S-phenyl, phenyl once or more than once substituted by halogen, -CF₃, C₁-C₆alkyl, -C₁-C₆ alkoxy, carboxy, -COO-C₁-C₆alkyl , whereby in case of a more than once substitution, the substituent can be the same or different;
R₁ is hydrogen, -C₁-C₆ alkoxy, -C₁-C₆ alkylthio, halogen or -NO₂, more preferably hydrogen or methoxy.
R₂ is hydrogen or -C₁-C₆ alkoxy, more preferably hydrogen or methoxy.
R₃ is NO₂.
R₄ is hydrogen, -C₁-C₆ alkoxy or halogen; more preferably hydrogen or methoxy.
R₅ is hydrogen, halogen, -C₁-C₆ alkoxy, -COOH; more preferably hydrogen, halogen, methoxy or-COOH.
R₆ is hydrogen.
R₇ is hydrogen.
Rₐ is methyl or ethyl.
R_{b} is methyl or ethyl.
R₈ is C₃-C₆alkyl.
R₉ is phenyl, mesityl, phenyl-O-phenyl, phenyl-S-phenyl, phenyl once or more than once substituted by halogen, -CF₃, C₁-C₆alkyl, -C₁-C₆ alkoxy, carboxy, -COO-C₁-C₆alkyl.
R₁₀ is hydrogen, C₁-C₆alkyl, more preferably hydrogen.
L and L' independently of one another are 1,3 phenylene or 1,4 phenylene wherein the phenylene linker is optionally substituted once or more than once by halogen, -CF₃, -C₁-C₆ alkyl, -C₁-C₆ alkoxy, carboxy , -COO-C₁-C₆alkyl, -CONH₂, -CON(C₁-C₆alkyl)₂, nitro; or L is naphthalene, biphenylene or phenylene-O-phenylene.

In a preferred embodiment the present invention provides a time temperature indicator comprising a compound of the formula I wherein
- R₁: is hydrogen, -C₁-C₆ alkoxy, -C₁-C₆ alkylthio, halogen or -NO₂,
- R₂: is hydrogen or -C₁-C₆ alkoxy;
- R₃: is NO₂;
- R₄: is hydrogen, -C₁-C₆ alkoxy or halogen;
- R₅ is: hydrogen, halogen, -C₁-C₆ alkoxy, -COOH;
- R₆: is hydrogen;
- R₇: is hydrogen;
- Rₐ: is methyl or ethyl;
- R_{b}: is methyl or ethyl;
- Y: is -CH₂-COO-R₈ or -CH₂-CO-N(R₁)-R₉; or -CH₂-CO-N(R₁₀)-L-N(R₁₀) CO-CH₂- or Y is -CH₂-CO-O-L'-O-CO-CH₂- wherein R₈ is C₃-C₆alkyl; R₉ is phenyl, mesityl, phenyl-O-phenyl, phenyl-S-phenyl, phenyl once or more than once substituted by halogen, -CF₃, C₁-C₆alkyl, -C₁-C₆ alkoxy, carboxy, -COO-C₁-C₆alkyl; R₁₀ is hydrogen, C₁-C₆alkyl, more preferably hydrogen. L and L' independently of one another are 1,3 phenylene or 1,4 phenylene wherein the phenylene linker is optionally substituted once or more than once by halogen, -CF₃, -C₁-C₆ alkyl, -C₁-C₆ alkoxy, carboxy , -COO-C₁-C₆alkyl, -CONH₂, -CON(C₁-C₆alkyl)₂, nitro; or L is naphthalene, biphenylene or phenylene-O-phenylene. (claim2)

In a more preferred embodiment the present invention provides a time temperature indicator comprising a compound of the formula I wherein
- R₁: is hydrogen or methoxy or methylthio;
- R₂: is hydrogen or methoxy;
- R₃: is nitro;
- R₄: is hydrogen or methoxy;
- R₅: is hydrogen, halogen, methoxy or -COOH;
- Rₐ: is methyl or ethyl;
- R_{b}: is methyl or ethyl;
- Y: is -CH₂-COO-R₈ or -CH₂-CO-N(R₁₀)-R₉; or -CH₂-CO-N(R₁₀)-L-N(R₁₀) CO-CH₂- or Y is -CH₂-CO-O-L'-O-CO-CH₂- wherein
R₈ is C₃-C₆alkyl;
R₉ is phenyl, mesityl, phenyl-O-phenyl, phenyl-S-phenyl ,phenyl once or more than once substituted by halogen, CF₃, C₁-C₆alkyl, -C₁-C₆ alkoxy, carboxy, -COO-C₁-C₆alkyl;
R₁₀ is hydrogen;
L and L' independently of one another are 1,3 phenylene or 1,4 phenylene wherein the phenylene linker is optionally substituted once or more than once by halogen, -CF₃, C₁-C₆alkyl, -C₁-C₆ alkoxy, carboxy , -COO-C₁-C₆alkyl, -CONH₂, -CON(C₁-C₆alkyl)₂, nitro; or L is naphthalene, biphenylene or phenylene-O-phenylene. (claim 3)

Most preferred according to the examples are:
R₂, R₄, R₅, R₆ and R₇ are hydrogen or R₆ and R₇ forms a phenyl ring;
R₉ is phenyl, phenyl-O-phenyl, phenyl-S-phenyl, mesityl, phenyl once or more than once substituted by halogen, -CF₃, C₁-C₆alkyl, methoxy, -COO-C₁-C₆alkyl.
L is 1,3 phenylene or 1,4 phenylene wherein the phenylene linker is optionally substituted once or more than once by halogen, -C₁-C₆ alkyl, -COO-C₁-C₆alkyl, nitro; or L is naphthalene or phenylene-O-phenylene.

In one embodiment the novel time-temperature indicator (TTI) system is based on indolenin based spiropyrans containing a N-acetylamido side chain. (claim 4)

In one embodiment the novel time-temperature indicator (TTI) system is based on indolenin based spiropyrans containing a N-acetylester side chain. (claim 5)

In one embodiment the novel time-temperature indicator (TTI) system is based on dimeric indolenin based spiropyrans wherein Y is-CH₂-CO-N(R₁₀)-L-N(R₁₀) CO-CH₂- (claim 6)

In one embodiment the novel time-temperature indicator (TTI) system is based on dimeric indolenin based spiropyrans wherein Y is -CH₂-CO-O-L'-O-CO-CH₂- (claim 7)

The following table shows the examples of compounds of the formula I wherein R2, R4, R5, R6 and R7 are hydrogen and Rb is methyl, R3 is nitro and Y is -CH₂-CO-N(R₁₀)-R₉.

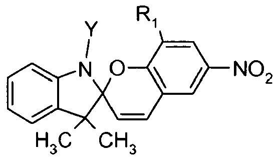

| Example | R9 | R10 | R1 |
|---|---|---|---|
| LF3453 | H | H | MeO |
| LF3459 | mesityl | H | MeO |
| LF3447 | ethyl | ethyl | MeO |
| LF3458 | methyl | methyl | MeO |
| LF3466 | pentyl | H | MeO |
| LF3453 | H | H | MeO |
| LF3472 | phenyl | H | H |
| LF3471 | phenyl | H | MeO |
| LF3451 | 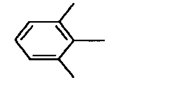 | H | MeO |
| LF3485 | 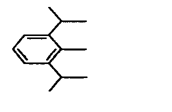 | H | MeO |
| LF3486 | 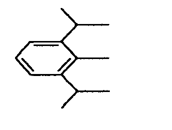 | H | H |
| LF3550 | 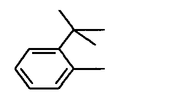 | H | MeO |
| LF3886 | 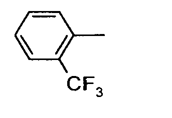 | H | MeO |
| LF3883 | 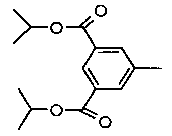 | H | MeO |

The following table shows examples of compounds of the formula I wherein R2, R4, R5, R6 and R7 are hydrogen, R3 is nitro, Ra and Rb is methyl, and Y is -CH₂-CO-NH(R₉)

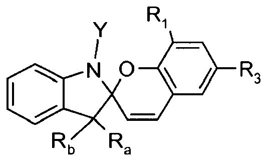

| | R9 | R1 | R3 | Ra |
|---|---|---|---|---|
| LF3837 | 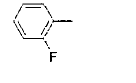 | MeO | NO₂ | Methyl |
| LF3838 | 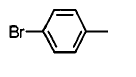 | MeO | NO₂ | Methyl |
| LF3843 | 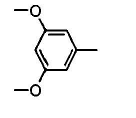 | MeO | NO₂ | Methyl |
| LF3847 | 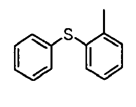 | MeO | NO₂ | Methyl |
| LF3848 | 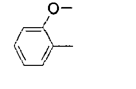 | MeO | NO₂ | Methyl |
| LF3849 | 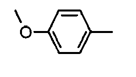 | MeO | NO₂ | Methyl |
| LF3997 | 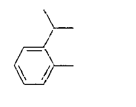 | EtO | NO₂ | Methyl |
| LF3998 | 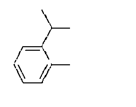 | MeS | NO₂ | Methyl |
| LF3999 | 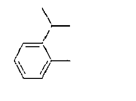 | Br | NO₂ | Methyl |
| LF4001 | 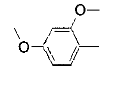 | MeO | NO₂ | Methyl |
| LF4005 | 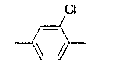 | MeO | NO₂ | Methyl |
| LF4009 | 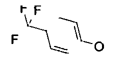 | MeO | NO₂ | Methyl |
| LF4021 | 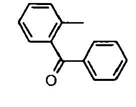 | MeO | NO₂ | Methyl |
| LF4022 | 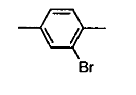 | MeO | NO₂ | Methyl |
| LF4026 | 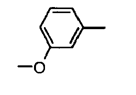 | MeO | NO₂ | Methyl |
| LF4028 | 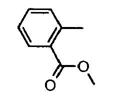 | MeO | NO₂ | Methyl |
| LF4031 | 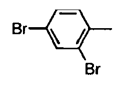 | MeO | NO₂ | Methyl |
| LF4035 | 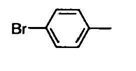 | EtO | NO₂ | Methyl |
| LF4036 | 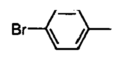 | MeS | NO₂ | Methyl |
| LF4037 | 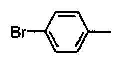 | Br | NO₂ | Methyl |
| LF4102 | 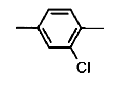 | EtO | NO₂ | Methyl |
| LF4103 | 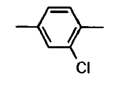 | Ph- | NO₂ | Methyl |
| LF4104 | 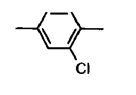 | Br | NO₂ | Methyl |
| LF4105 | 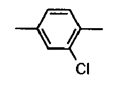 | Cl | NO₂ | Methyl |
| LF4106 | 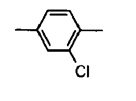 | MeS | NO₂ | Methyl |
| LF4107 | 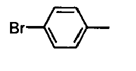 | Cl | NO₂ | Methyl |
| LF4108 | 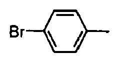 | Ph | NO₂ | Methyl |

The following example is a compound of formula I wherein R1 is MeO, R2 is H, R3 is nitro, R4 is H, Ra and Rb are methyl, R5 is H, R6 and R7 form together a phenyl ring, and Y is - CH₂-CO-NH-phenyl.

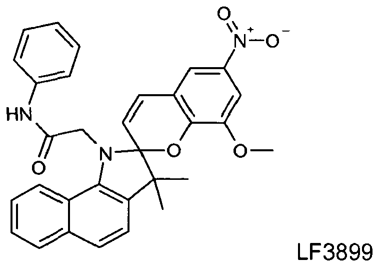

The following table shows examples of compounds of the formula I wherein R2, R4, R5, R6 and R7 are hydrogen, R3 is nitro and Rb is methyl, and Y is -CH₂-CO-N(H)-L-N(H) CO-CH₂-

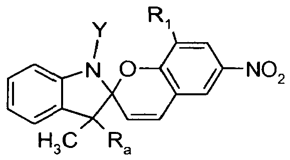

| Example | R1 | L | Ra |
|---|---|---|---|
| LF3482 | MeO | 1,4-phenylene | methyl |
| LF3564 | H | 1,4-phenylene | methyl |
| LF3644 | H | 1,4-phenylene | ethyl |
| LF3643 | MeO | 1,4-phenylene | ethyl |
| LF3658 | MeO | 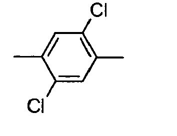 | methyl |
| LF3659 | H | 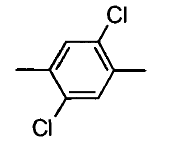 | methyl |
| LF3668 | H | 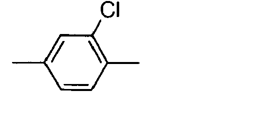 | methyl |
| LF3733 | MeO | 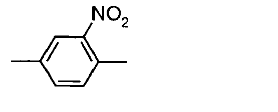 | methyl |
| LF3724 | MeO | 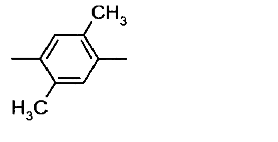 | methyl |
| LF3725 | H | 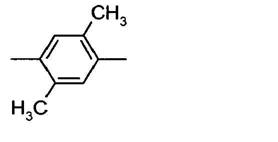 | methyl |
| LF3671 | MeO | 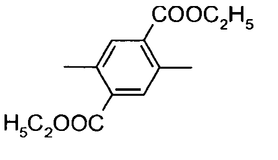 | methyl |
| LF3672 | H | 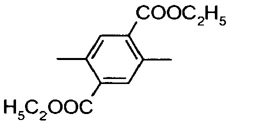 | methyl |
| LF3568 | H | 1,3-phenylene | methyl |
| LF3567 | MeO | 1,3-phenylene | methyl |
| LF3570 | MeO | 1,3-phenylene | ethyl |
| LF3571 | H | 1,3-phenylene | ethyl |
| LF3592 | MeO | 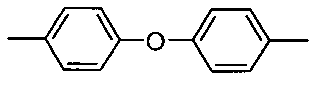 | methyl |
| LF3594 | H | 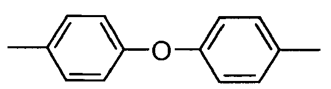 | methyl |
| LF3729 | MeO | Napthalene | methyl |

The following table shows the examples of compounds of the formula I wherein R2, R4, R5, R6 and R7 are hydrogen and R3 is nitro and Y is -CH₂-COO-R₈

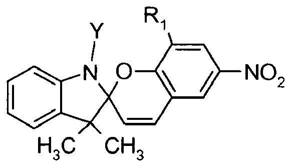

| | R8 | R1 |
|---|---|---|
| LF3608 | methyl | MeO |
| LF3475 | ethyl | MeO |
| LF3476 | ethyl | H |
| LF3561 | H | MeO |
| LF 3720 | propyl | MeO |
| LF 3721 | i-propyl | MeO |
| LF4032 | i-propyl | EtO |
| LF4033 | i-propyl | MeS |

The following example is a compound of formula I wherein R1 is MeO, R2 is H, R3 is nitro, R4 is H, Ra and Rb are methyl, R5 is H, R6 and R7 form together a phenyl ring, and Y is - CH₂-COOEt.

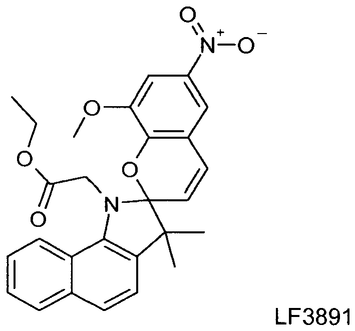

The following table shows the examples of compounds of the formula I wherein R2, R4, R5, R6 and R7 are hydrogen and R3 is nitro and Y is -CH₂-CO-O-L'-O-CO-CH₂-

| | R8 | R1 | R3 | L | Ra / Rb |
|---|---|---|---|---|---|
| LF3684 | | MeO | NO₂ | 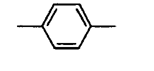 | Methyl |
| LF3689 | | MeO | NO₂ | 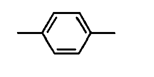 | Ethyl / Methyl |
| LF3777 | | MeO | NO₂ | 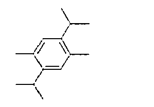 | Methyl |
| LF3878 | | MeO | NO₂ | -CH₂-CH₂- | Methyl |

### Preparation

The compounds are prepared according to the general scheme below.

### Indolenin based Spiropyrans containing a N-Acetylamido Side chain

Indolenin based Spiropyrans containing a N-Acetylamido side chain are made using a 3 step synthesis if the starting Bromo- (or Chloro-)-acetyl amid is not commercially available. 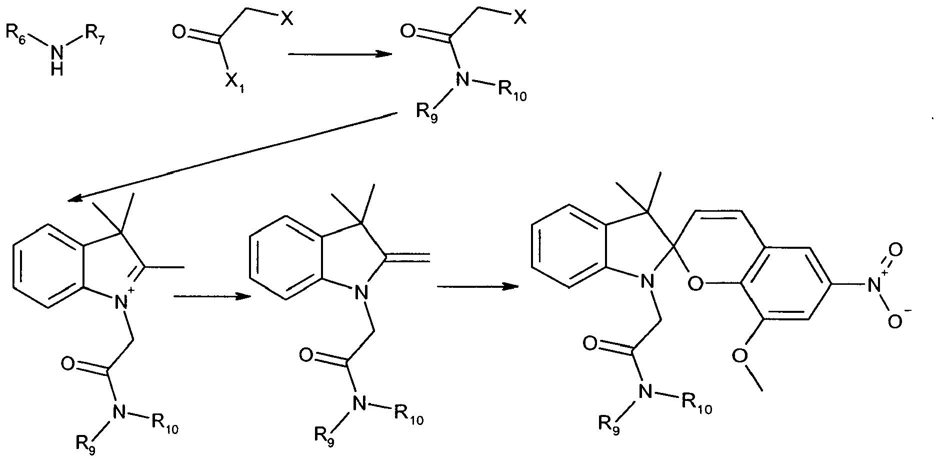 Indolenin based Spiropyrans containing an Acetylester side chain are made using a 2 step synthesis. A big range of Bromo- or Chloro-acetylesters are commercially available.

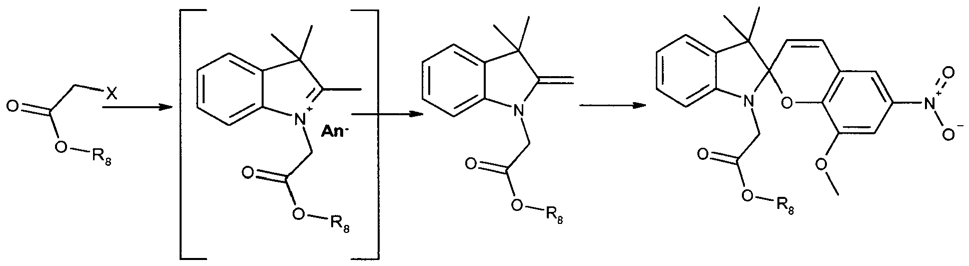

The inventive TTI relies on a spiroaromatic compound which is reversibly photochromic. By virtue of its photochromic properties, the indicator compound can undergo photo-induced coloration by irradiation with photons of a specific energy range (conversion of the second isomeric form into the first isomeric form), the coloration being followed by a time- and temperature-dependent decoloration (conversion of the first isomeric form into the second isomeric form). The coloration of the indicator compound can take place at a defined timepoint, preferably, for example, immediately after printing onto a substrate, which is especially the packaging of a perishable material.

For example, the initially colorless indicator compound is irradiated with UV light or near-UV light, whereupon an isomerization within the indicator compound (conversion of the second isomeric form into the first isomeric form) and an associated indicator compound coloration takes place. Such a photo-induced isomerization then proceeds as a function of time and temperature in the other direction again, so that the indicator is successively decolorized.

In each spiropyran compound exist at least two distinct isomeric forms, at least one open form and at least one cyclic isomeric form that can be converted into each other by valence isomerization:

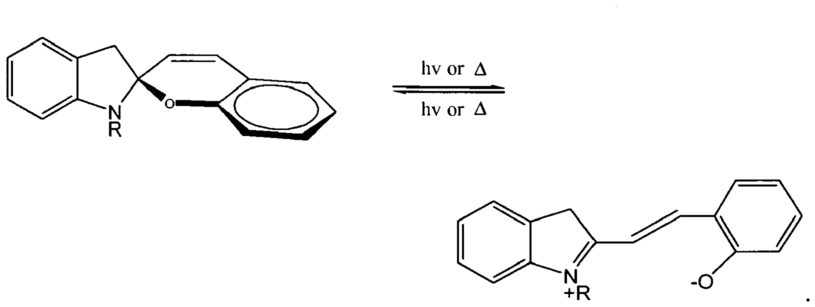

In the colored state only negligible effect is found to any stimulus other than temperature.

In another aspect of the present invention, there is provided a method for manufacturing a time-temperature indicator comprising at least one of the spiroaromatic indicator compounds of the formula I; said method comprising the steps of
(a) introducing into a matrix or atop a matrix a spiropyran indicator of the formula I as defined in claim 1 or in claim 8 and
(b) converting the spiropyran indicator from an original stable state into a metastable state by a process selected from photonic induction, thermal induction, pressure induction, electrical induction, or chemical induction, preferably photonic induction;
(c) optionally applying a protector film. (claim 9)

The metastable state of the compounds used with the TTIs of the present invention may be achieved by one of the various stimuli mentioned hereinabove. In one embodiment, the metastable state is generated by photonic induction, wherein a matrix embedded with the substance is positioned or passed under a light source, emitting light of a wavelength and intensity suitable for photoexcitation, such as UV. The exposure to the light is terminated when the embedded substance changes its color to a color indicative of the formation of the metastable state at a pre-fixed quantity.

In another embodiment, the metastable state is achieved by pressure induction. In this procedure, the matrix embedded with and/or atop the substance is passed between two bodies, such as metal rolls, which apply pressure onto the surface of the matrix thereby inducing the formation of the metastable state. By adjusting the time and pressure imparted by the bodies to the active material, it is possible to control the degree of conversion from a stable state to a metastable state in the TTI active matrix.

In yet another embodiment, the metastable state is achieved by thermal induction. In this particular induction process, the matrix embedded with the substance to be induced is heated to temperatures normally below the melting point of said substance. The heat may be applied by any method known such as, but not limited to, a thermal transfer printing head. In one specific case, the heat is applied to the matrix while being passed through two heated metal rolls. In this case, the pressure applied to the surface is not capable itself of inducing the formation of the metastable state, but serves merely to ensure controlled thermal contact between the heaters and the sample. The metastable state is achieved as a result of the heat transfer from the heaters, i.e., the metal rolls, which are in contact with the matrix and the matrix itself.

However, there may be instances where the use of any combination of pressure, light and thermal inductions may be desired or necessary. It is therefore, a further embodiment of the present invention, to achieve the metastable state of the substances to be used with the TTIs of the present invention, by a combination of stimuli.

The support matrix used in the present invention may be a polymer such as PVC, PMMA, PEO polypropylene, polyethylene, all kinds of paper, all kinds of printing media or the like or any glass-like film. The active indicator may be introduced into and/or atop a matrix substrate such as polymers, glass, metals, paper, and the like, and may take on in the matrix any form that may permit reversibility of the induced chromic process. Such forms may be or result from indicator-doping of the matrix, sol-gel embedment of the indicator in the matrix, embedment of the indicator as small crystallites, solid solution and the like.

Some of the spiropyrans of the examples are already described.

The Japanese Publication JP62242686 (1987) discloses N-substituted spiropyrans having a N-acetylamido side chain -CH₂-CON(alkyl)₂ or -CH₂-CONH₂ or -CH₂-CONH(alkyl).

M.A. Galbertshtam describes in Chemistry of heterocyclic compounds, Vol 13, 1977, pages 1309-1313 the photochromic properties of some N-substituted spiropyrans having a N-acetylester side chain. Specifically disclosed are carb-ethoxymethyl side chains -CH₂-COO Et.

The spiropyrans are not described in the above references as being used to prepare a time temperature indicator.

Therefore the invention relates to the use of spiropyrans of the formula I' for manufacturing a time temperature indicator

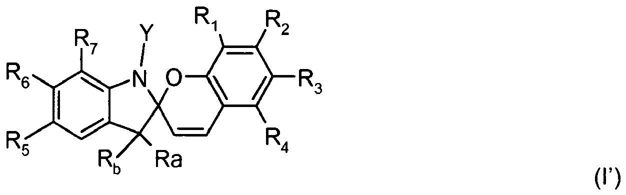

Wherein
- R₁: is hydrogen, -C₁-C₁₈ alkoxy, -C₁-C₁₈ alkylthio, -C₁-C₁₈ alkyl-SO-, -C₁-C₁₈ alkyl-SO₂-, phenylthio, phenyl, halogen, -C₁-C₁₈ alkyl or -NO₂;
- R₂: is hydrogen or -C₁-C₁₈ alkoxy;
- R₃: is NO₂ or halogen;
- R₄: is hydrogen, -C₁-C₁₈ alkoxy or halogen;
- R₅: is hydrogen, halogen, -C₁-C₁₈ alkoxy, -COOH, -COO-C₁-C₁₈alkyl, -CF₃ or phenyl;
- R₆: is hydrogen or R₆ and R₇ form together a phenyl ring;
- R₇: is hydrogen;
- Rₐ: is hydrogen or -C₁-C₆ alkyl;
- R_{b}: is hydrogen or -C₁-C₆ alkyl, or together with Rₐ form a 5-6 membered ring;
- Y: is -CH₂-COO-R₈ or -CH₂-CO-N(R₁₀)-R₉; or -CH₂-CO-N(R₁₀)-L-N(R₁₀) CO-CH₂- or Y is -CH₂-CO-O-L'-O-CO-CH₂-wherein
R₈ is hydrogen, C₁-C₁₈alkyl;
R₉ is phenyl, mesityl, naphthyl, or higher annelated aromatic systems, phenyl-O-phenyl, phenyl-S-phenyl, or phenyl, naphthyl or the higher annelated aromatic system is once or more than once substituted by halogen, -CF₃, C₁-C₆alkyl, -C₁-C₆ alkoxy, carboxy,-COO-C₁-C₆alkyl , CN, NO₂, N(R₁₁)₂, S-R₁₁ (SO-R₁₁, SO₂-R₁₁), CO-R₁₁, CO-N(R₁₁)₂ (R11=C1-C18alkyl, aryl, substituted aryl) whereby in case of a more than once substitution, the substituent can be the same or different;
R₁₀ is hydrogen, C₁-C₁₈alkyl;
L is 1,3 phenylene or 1,4 phenylene wherein the phenylene linker is optionally substituted by once or more than once by halogen, -CF₃, C₁-C₁₈alkyl, -C₁-C₁₈ alkoxy, carboxy , -COO-C₁-C₁₈alkyl, -CONH₂, -CON(C₁-C₁₈alkyl)₂, nitro; or L is naphthalene, biphenylene or phenylene-O-phenylene wherein the naphthalene, biphenylene or phenylene-O-phenylene linker is optionally substituted once or more than once by halogen, -CF₃, C₁-C₁₈alkyl, -C₁-C₁₈ alkoxy, carboxy , -COO-C₁-C₁₈alkyl, -CONH₂,-CON(C₁-C₁₈alkyl)₂, nitro.
L' is 1,3 phenylene or 1,4 phenylene wherein the phenylene linker is optionally substituted by once or more than once by halogen, -CF₃, C₁-C₁₈alkyl, -C₁-C₁₈ alkoxy, carboxy , -COO-C₁-C₁₈alkyl, -CONH₂, -CON(C₁-C₁₈alkyl)₂, nitro; or L is naphthalene, biphenylene or phenylene-O-phenylene wherein the naphthalene, biphenylene or phenylene-O-phenylene linker is optionally substituted once or more than once by halogen, -CF₃, C₁-C₁₈alkyl, -C₁-C₁₈ alkoxy, carboxy , -COO-C₁-C₁₈alkyl, -CONH₂,-CON(C₁-C₁₈alkyl)₂, nitro. (claim 8)

In another embodiment, the present invention also relates to a method of determining the time temperature history of perishable goods, which method comprises the following steps:
a) printing onto a substrate a time-temperature integrator which comprises at least one spiroaromatic indicator compound as defined in claim 1 or in claim 8;
b) activating the indicator, preferably by photo-induced coloration
c) optionally applying a protector that prevents renewed photo-induced coloration of the indicator, and
d) determining the degree of time- or temperature-induced decoloration and, taking account of the degree of decoloration, the quality of the product. (claim 10)

In a preferred embodiment of the present invention, the indicator compound as the active material of the time-temperature indicator is provided in an ink formulation, which is directly printed onto said packaging material or label.

Any of the printing methods known in the art, e.g., ink jet printing, flexo printing, laser printing, offset printing, intaglio printing, screen printing and the like.

In another embodiment, the indicator compound is part of a thermal transfer (TTR) ink composition and is transferred to the printed surface by applying heat to the TTR layer.

When ink-jet printing is used, the procedure is advantageously as follows:

In Step a), a time-temperature integrator comprising at least one spiroaromatic indicator compound as defined above, is applied by means of ink-jet printing to the substrate, especially to the packaging of ageing- and temperature-sensitive products or to labels that are applied to the packaging.

In a preferred embodiment, in Step a) it is possible additionally to apply, by means of ink-jet printing, a reference scale which reproduces the change in the color of the indicator as a function of time, and it is possible to apply, preferably in black ink, further text (or information), such as an expiry date, product identification, weight, contents etc.

Step a) is followed by Step b), activation, especially photo-induced coloration of the indicator compound. The photo-induced curing of the binder advantageously includes the photo-induced coloration of the indicator.

If desired, following Step b), an irreversible photo-sensitive indicator can be applied as tamper-proofing in the form of a covering over the time-temperature integrator. Suitable irreversible indicators include, for example, pyrrole derivatives, such as 2-phenyl-di(2-pyrrole)methane. Such a material turns irreversibly red when it is exposed to UV light.

Step c) is followed by the application of a protector, especially a color filter, which prevents renewed photo-induced coloration of the reversible indicator. In the case of UV-sensitive indicators, there come into consideration yellow filters, which are permeable only to light having typical wavelengths that are longer than 430 nm. Advantageously the protective film, that is to say the color filter, can likewise be applied by means of ink-jet printing.

Suitable filters are disclosed in the International application EP2007/060987, filed October 16, 2007. Disclosed therein is a composition comprising at least one ultraviolet light and/or visible light absorbing layer which is adhered to an underlying layer containing a photochromic colorant, which photo chromic colorant is activated by exposure to UV light to undergo a reversible color change, which color reversion occurs at a rate that is dependent on temperature, wherein the light absorbing layer comprises a binder, from 1 to 60% by weight based on the total weight of the layer of an ultraviolet light absorber selected from the group consisting of hydroxyphenylbenzotriazole, benzophenone, benzoxazone, α-cyanoacrylate, oxanilide, tris-aryl-s-triazine, formamidine, cinnamate, malonate, benzilidene, salicylate and benzoate ultraviolet light absorbers.

The time-temperature clock can be started at a defined desired timepoint. Decoloration is preferred for consideration according to the invention, but the use of an indicator in which the coloration process forms the basis of the time-temperature clock is also conceivable.

The actual determination of the quality of ageing- or temperature-sensitive products is preceded by the activation of the indicator in Step b). At a later timepoint, the degree of time- or temperature-induced decoloration is then measured and the quality of the product is inferred therefrom. When an evaluation is made with the aid of the human eye, it may be advantageous to arrange e.g. alongside or below the substrate a reference scale which allocates a certain quality grade, a certain timepoint etc. to a certain degree of decoloration. When the quality of the product is determined by evaluating the degree of decoloration or coloration, it is therefore preferred to use a reference scale.

The substrate can simultaneously form the packaging material for the perishable products or it can be applied to the packaging material, for example in the form of a label.

By means of a reference scale printed with the time-temperature integrator, absolute determination of quality grades is possible. The time-temperature integrator and the reference scale are advantageously arranged on a light-colored substrate in order to facilitate reading.

Suitable substrate materials are both inorganic and organic materials, preferably those known from conventional layer and packaging techniques. There may be mentioned by way of example polymers, glass, metals, paper, cardboard etc.

The substrates are suitable for use as packaging materials for the goods and or for attachment thereto by any method known. It should be understood, that the indicators of the present invention may also be applicable to and used in the food industry, and essentially be similarly effective to other goods that may be used in the pharmaceutical or medical fields.

Another embodiment of the present invention concerns a packaging material or a label that comprises a time-temperature indicator as described above.

In yet another embodiment, the present invention also relates to a high molecular weight material that comprises at least one spiroaromatic indicator as described above.

The high molecular weight organic material may be of natural or synthetic origin and generally has a molecular weight in the range of from 10³ to 10⁸ g/mol. It may be, for example, a natural resin or a drying oil, rubber or casein, or a modified natural material, such as chlorinated rubber, an oil-modified alkyd resin, viscose, a cellulose ether or ester, such as cellulose acetate, cellulose propionate, cellulose acetobutyrate or nitrocellulose, but especially a totally synthetic organic polymer (thermosetting plastics and thermoplastics), as are obtained by polymerisation, polycondensation or polyaddition, for example polyolefins, such as polyethylene, polypropylene or polyisobutylene, substituted polyolefins, such as polymerisation products of vinyl chloride, vinyl acetate, styrene, acrylonitrile, acrylic acid esters and/or methacrylic acid esters or butadiene, and copolymerisation products of the mentioned monomers, especially ABS or EVA. From the group of the polyaddition resins and polycondensation resins there may be mentioned the condensation products of formaldehyde with phenols, so-called phenoplasts, and the condensation products of formaldehyde with urea, thiourea and melamine, so-called aminoplasts, the polyesters used as surface-coating resins, either saturated, such as alkyd resins, or unsaturated, such as maleic resins, also linear polyesters and polyamides or silicones. The mentioned high molecular weight compounds may be present individually or in mixtures, in the form of plastic compositions or melts. They may also be present in the form of their monomers or in the polymerised state in dissolved form as film-forming agents or binders for surface-coatings or printing inks, such as boiled linseed oil, nitrocellulose, alkyd resins, melamine resins, ureaformaldehyde resins or acrylic resins.

In order to better understand the present invention and to see how it may be carried out in practice, preferred embodiments will now be described, by way of non-limiting examples.

### Examples

### General Synthesis

### 1. N-(N',N'-Diethylaminoacetamido)-2-methylene-3,3-dimethyl-indolenin

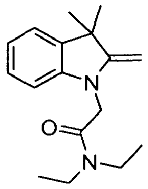

2-Chloro-N,N-diethylacetamide (25g / 0,162 mol) and 2,3,3-Trimethylindolenin (10,5g / 0,06mol) were mixed together, 0,2g Potassium iodid is added as catalyst. The mixture is heated to 100°C for 24h. After cooling to room temperature 50ml of water and Toluene are added, and extracted. The organic phase was discarded. 50ml of 0,2m NaOH was added to the aqueous phase, the pH increased from around 2 to 11. The aqueous phase was extracted twice with Dichloromethane, the organic phase was dried using sodium sulphate. The solvent was removed by means of a Rotavap. 17g of a yellowish oil was recovered.

### 2. N-(N',N'-Diethylaminoacetamido)-3,3-dimethyl-indolenin-nitro-methoxy-spiropyran

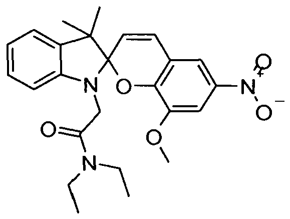

12,5g Nitro-o-vanillin (0,062mol) was dissolved in 150ml Ethanol at 60°C, in a second flask also 17g N-(N',N'-Diethylaminoacetamido)-2-methylene-3,3-dimethyl-indolenin (0,062mol) were dissolved in 150ml Ethanol at 60°C. Both solutions were put together at 60°, after 1 min the solution was cooled very slowly to room temperature with strong stirring. The precipitate was filtered, washed 3 times with Ethanol and dried. 14g (50%) of a brownish powder was obtained.

### 3. Bromoacteyl-2,4,6-trimethylanilid

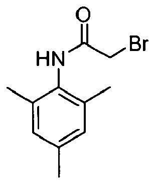

20.6g (0,05mol) of bromoacetyl bromide was dissolved in 175ml Acetonitrile, afterwards 7g potassium carbonate (dry, 0,05mol) was added. To the resulting white suspension 13.7g 2,4,6-Trimethylanilin (0.1 mol) in 125ml Acetonitrile was added in 30 min at room temperature under strong agitation. After 3h the precipitate was filtered and washed 4 times with Acetonitrile. The filtrate was evaporated till about 2/3 of the volume and chilled subsequently to 5°C over night. The white crystals were collected, the yield was 11,7g (46%) of Bromoacteyl-2,4,6-trimethylanilid.

### 4. N-(N-2,4,6-trimethylanilidoacetamid)-2-methylene-3,3-dimethyl-indolenin

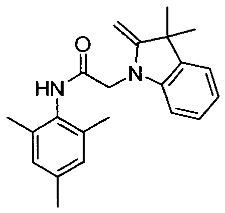

4g (0.016mol) Bromoacteyl-2,4,6-trimethylanilid, 2.5g (0.016mol) 2,3,3-Trimethylindolenin and 1.07g (0.008mol) potassium carbonate were mixed in 50ml Acetonitrile and subsequently refluxed for 26h. After cooling the precipitate was removed by filtration and the solvent was evaporated till dryness. 5.2g of a yellowish oil was obtained which is mostly the wanted product with some of 2,3,3-Trimethylindolenin.

### 5. N-(N-2,4,6-trimethylanilidoacetamid)-3,3-dimethyl-indolenin-nitro-methoxy-spiropyran

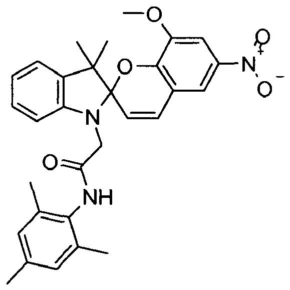

3g Nitro-o-vanillin (0,016mol) was dissolved in 150ml Ethanol at 60°C, in a second flask also 5.2g (0,016mol) N-(N-2,4,6-trimethylanilidoacetamid)-2-methylene-3,3-dimethyl-indolenin were dissolved in 150ml Ethanol at 60°C. Both solutions were put together at 60°, after 1 min the solution was cooled very slowly to room temperature with strong stirring. The precipitate was filtered, washed 3 times with Ethanol and dried. 4.5g (57%) of a beige powder was obtained.

The compounds of the above Table were made similarly:

### General Synthesis

### 1. N-(Ethylacetate)-2-methylene-3,3-dimethyl-indolenin

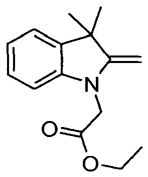

44.8g (0.26mol) bromoacetic acid ethylester were put into a flask, 14.1g (0.0087mol) 2,3,3,-Trimethylindolenin was added and heated to 80°C under stirring. The heating was continued 22h. After cooling to room temperature 200ml Water was added and extracted with 200 ml Dichloromethane. 45ml 2m sodium hydroxide solution was added to the aqueous phase to shift the pH value from around 1 to 11. The solution was extracted twice with 200ml Dichloromethane, the organic phase was dried with sodium sulphate and evaporated to dryness. 11.8g of a yellowish oil was recovered, which contained the wanted product and some not reacted 2,3,3,-Trimethylindolenin.

### 2. N-(Ethyl acetate)-3,3-dimethyl-indolenin-nitro-methoxy-spiropyran

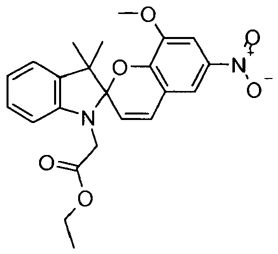

4.7g (0,024mol) Nitro-o-vanillin was dissolved in 50ml Ethanol at 60°C, in a second flask also 5.9g (0,024mol) N-(Ethyl acetate)-2-methylene-3,3-dimethyl-indolenin was dissolved in 50ml Ethanol at 60°C. Both solutions were put together at 60°, after 1 min the solution was cooled very slowly to room temperature with strong stirring. The precipitate was filtered, washed 3 times with Ethanol and dried. 4.4g (43%) of a brownish powder was obtained.

The compounds of the above Table were made similarly:

### Bleaching behaviour:

| | | | | (L² + a² + b²)^{0.5} | |
|---|---|---|---|---|---|
| Compound | (L² + a² + b²)^{0.5} uncharged | Charge conditions | Time hrs | | |
| 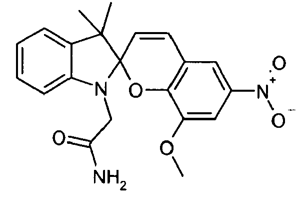 | 91.73 | 10s | 0 | 61.7 | LF3453 |
| | | | 24 | 71.3 | |
| | | | 48 | 72.1 | |
| | | | 192 | 75.4 | |
| 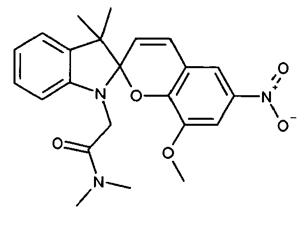 | 93.86 | 10s | 0 | 66.8 | LF3458 |
| | | | 24 | 80.3 | |
| | | | 48 | 83.0 | |
| | | | 120 | 85.9 | |
| 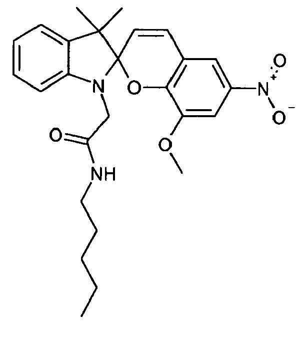 | 88.95 | | 0 | 55.5 | LF3466 |
| | | | 24 | 57.8 | |
| | | | 48 | 60.3 | |
| | | | 72 | 60.8 | |
| | | | 96 | 62.2 | |
| | | | 172 | 64.0 | |
| 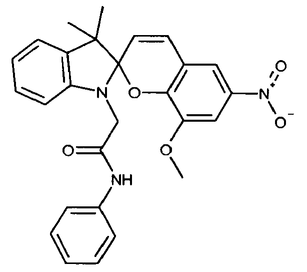 | 93.48 | | 0 | 69.8 | LF3471 |
| | | | 24 | 76.9 | |
| | | | 48 | 80.3 | |
| | | | 72 | 81.8 | |
| | | | 96 | 82.7 | |
| 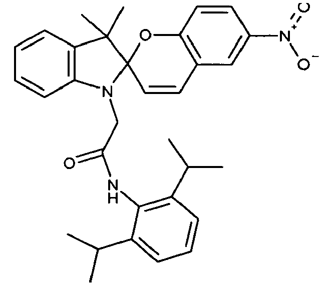 | 93.69 | | 0 | 69.4 | LF3486 |
| | | | 24 | 69.9 | |
| | | | 48 | 70.6 | |
| | | | 96 | 71.9 | |
| | | | 172 | 73.7 | |
| 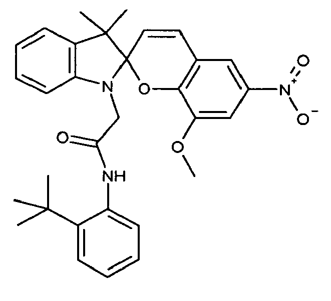 | 96.32 | | 0 | 54.4 | LF3550 |
| | | | 24 | 69.6 | |
| | | | 48 | 74.0 | |
| | | | 96 | 76.6 | |
| | | | 120 | 79.1 | |
| | | | 172 | 82.8 | |
| 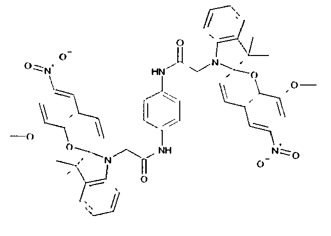 | 80.60 | | 0 | 50.3 | LF3482 |
| | | | 24 | 52.2 | |
| | | | 96 | 52.4 | |
| | | | 196 | 52.6 | |
| 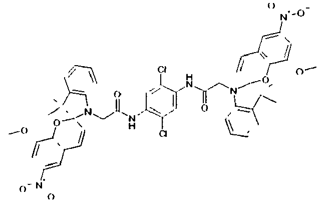 | 89.19 | | 0 | 53.0 | LF3658 |
| | | | 24 | 56.0 | |
| | | | 48 | 56.2 | |
| | | | 72 | 57.1 | |
| | | | 144 | 58.0 | |
| | | | 196 | 58.6 | |
| 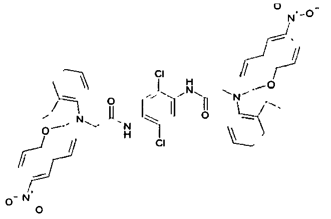 | 90.62 | | 0 | 72.0 | LF3659 |
| | | | 24 | 75.3 | |
| | | | 48 | 75.8 | |
| | | | 144 | 76.2 | |
| | | | 240 | 78.2 | |
| 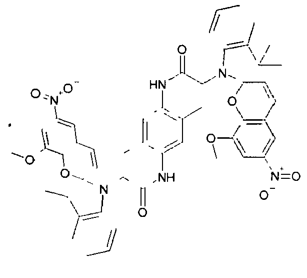 | 93.29 | | 0 | 53.1 | LF3724 |
| | | | 24 | 55.9 | |
| | | | 48 | 57.3 | |
| | | | 96 | 59.0 | |
| | | | 240 | 60.8 | |
| 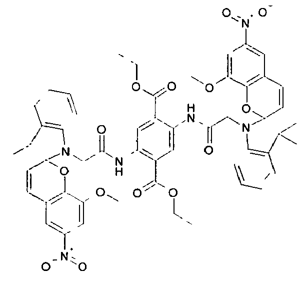 | 92.64 | | 0 | 64.0 | LF3671 |
| | | | 24 | 70.2 | |
| | | | 48 | 72.2 | |
| | | | 144 | 75.6 | |
| 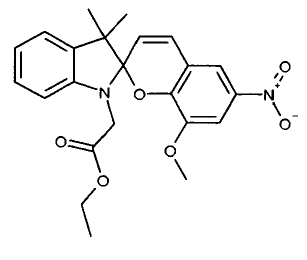 | 91.08 | | 0 | 52.9 | LF3475 |
| | | | 24 | 57.5 | |
| | | | 48 | 62.3 | |
| | | | 72 | 65.8 | |
| | | | 96 | 68.9 | |
| | | | 192 | 71.4 | |
| | | | 264 | 73.2 | |
| 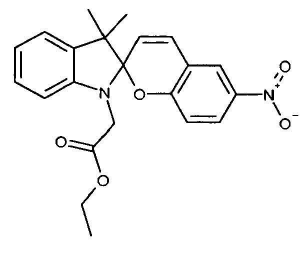 | 93.26 | | 0 | 71.5 | LF3476 |
| | | | 24 | 74.1 | |
| | | | 48 | 79.9 | |
| | | | 72 | 82.5 | |
| | | | 216 | 87.7 | |
| 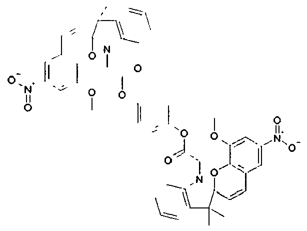 | 81.84 | | 0 | 52.1 | LF3684 |
| | | | 24 | 60.2 | |
| | | | 48 | 61.7 | |
| | | | 96 | 64.5 | |
| | | | 240 | 68.1 | |
| 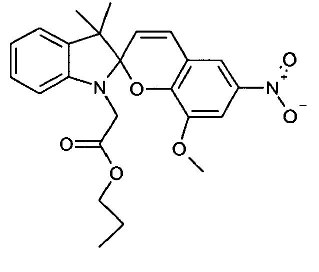 | 93.97 | | 0 | 55.3 | LF3720 |
| | | | 24 | 62.1 | |
| | | | 48 | 63.9 | |
| | | | 72 | 64.9 | |
| | | | 144 | 67.4 | |
| | | | 240 | 68.8 | |
| 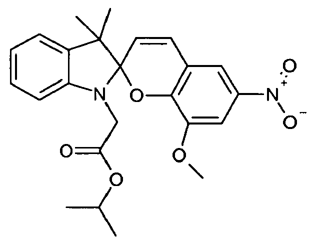 | 90.58 | | 0 | 53.0 | LF3721 |
| | | | 24 | 62.5 | |
| | | | 48 | 66.4 | |
| | | | 96 | 71.6 | |
| | | | 240 | 76.2 | |

| Compound | L-value uncharged | Charge conditions | Time hrs | L-value | Nr. |
|---|---|---|---|---|---|
| 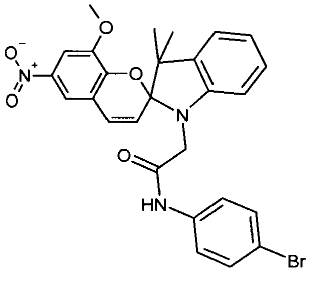 | 90 | 10s | 0 | 63 | LF3838 |
| | | | 25 | 70.3 | |
| | | | 50 | 73.4 | |
| | | | 119 | 75.7 | |
| | | | 167 | 77.5 | |
| 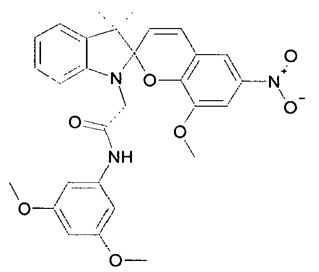 | 90 | 10s | 0 | 57.7 | LF3843 |
| | | | 25 | 65.1 | |
| | | | 52 | 68.3 | |
| | | | 77 | 70.7 | |
| | | | 96 | 71.6 | |
| | | | 218 | 74.8 | |
| 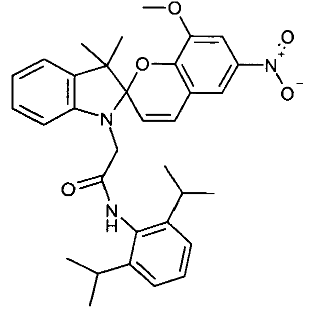 | 93.7 | 10s | 0 | 62.5 | LF3485/2 |
| | | | 25 | 70.5 | |
| | | | 52 | 73 | |
| | | | 73 | 74.1 | |
| | | | 96 | 75.1 | |
| | | | 193 | 76.9 | |
| 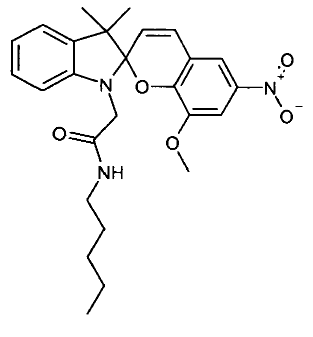 | 88.9 | 4s | 0 | 36.5 | LF3466 |
| | | | 26 | 47.9 | |
| | | | 50 | 53.8 | |
| | | | 73 | 55.9 | |
| | | | 97 | 59.3 | |
| | | | 194 | 61.4 | |
| | | | 265 | 63.7 | |
| 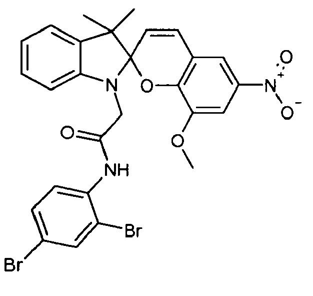 | 86.9 | 10s | 0 | 41.5 | LF4031 |
| | | | 26 | 52.9 | |
| | | | 98 | 62.4 | |
| | | | 124 | 63.7 | |
| | | | 195 | 65.8 | |
| | | | 293 | 67.1 | |
| 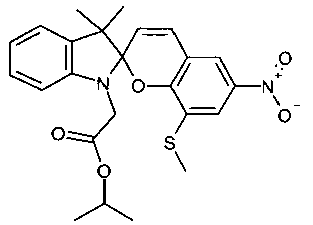 | 91.2 | 10s | 0 | 49 | LF4033 |
| | | | 25 | 68.1 | |
| | | | 53 | 71.8 | |
| | | | 123 | 74.5 | |
| | | | 167 | 75.9 | |

## Claims

1. A time temperature indicator comprising at least one spiropyran indicator of formula (I) 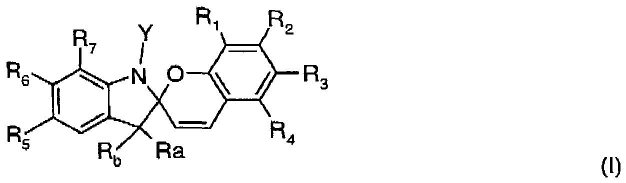 wherein
R₁ is hydrogen, -C₁-C₁₈ alkoxy, -C₁-C₁₈ alkylthio, -C₁-C₁₀ alkyl-SO-, -C₁-C₁₈ alkyl-SO₂-, phenylthio, phenyl, halogen, -C₁-C₁₈ alkyl or -NO₂;
R₂ is hydrogen or -C₁-C₁₈ alkoxy;
R₃ is NO₂ or halogen;
R₄ is hydrogen, -C₁-C₁₈ alkoxy or halogen;
R₅ is hydrogen, halogen, -C₁-C₁₈ alkoxy, -COOH, -COO-C₁-C₁₈alkyl, -CF₃ or phenyl;
R₆ is hydrogen or R₆ and R₇ form together a phenyl ring;
R₇ is hydrogen;
Rₐ is hydrogen or -C₁-C₆ alkyl;
R_{b} is hydrogen or -C₁-C₆ alkyl, or together with Rₐ form a 5-6 membered ring;
Y is -CH₂-COO-R₈ or -CH₂-CO-N(R₁₀)-R₉; or -CH₂-CO-N(R₁₀-L-N(R₁₀) CO-CH₂- or Y is -CH₂-CO-O-L'-O-CO-CH₂-
wherein
R₆ is hydrogen, C₃-C₁₈alkyl or R₈ is ethyl with the proviso that R₆ and R₇ form together a phenyl ring;
R₉ is phenyl, mesityl, or a higher than naphthyl annelated aromatic system, phenyl-O-phenyl, phenyl-S-phenyl, or phenyl, or the higher than naphthyl annelated aromatic system is once or more than once substituted by halogen, -CF₃, C₁-C₆alkyl, -C₁-C₆ alkoxy, carboxy, -COO-C₁-C₆alkyl , CN, NO₂, N(R₁₁)₂, S-R₁₁ (SO-R₁₁, SO₂-R₁₁), CO-R₁₁, CO-N(R₁₁)₂ (R11=C1-C18alkyl, aryl, substituted aryl) whereby in case of a more than once substitution, the substituent can be the same or different;
R₁₀ is hydrogen, C₁-C₁₈alkyl;
L is 1,3 phenylene or 1,4 phenylene wherein the phenylene linker is optionally substituted by once or more than once by halogen, -CF₃, C₁-C₁₈alkyl, -C₁-C₁₈, alkoxy, carboxy , -COO-C₁-C₁₆alkyl, -CONH₂, -CON(C₁-C₁₈alkyl)₂, nitro; or L is naphthalene, biphenylene or phenylene-O-phenylene wherein the naphthalene, biphenylene or phenylene-O-phenylene linker is optionally substituted once or more than once by halogen, -CF₃, C₁-C₁₈alkyl, -C₁-C₁₈ alkoxy, carboxy , -COO-C₁-C₁₈alkyl, -CONH₂,-CON(C₁-C₁₈alkyl)₂, nitro.
L' is 1,3 phenylene or 1,4 phenylene wherein the phenylene linker is optionally substituted by once or more than once by halogen, -CF₃, C₁-C₁₈alkyl, -C₁-C₁₈ alkoxy, carboxy , -COO-C₁C₁₈alkyl, -CONH₂, -CON(C₁-C₁₈alkyl)₂, nitro; or L is naphthalene, biphenylene or phenylene-O-phenylene wherein the naphthalene, biphenylene or phenylene-O-phenylene linker is optionally substituted once or more than once by halogen, -CF₃, C₁-C₁₈alkyl, -C₁-C₁₈ alkoxy, carboxy , -COO-C₁-C₁₈alkyl, -CONH₂,-CON(C₁-C₁₈alkyl)₂, nitro.

2. A time temperature indicator according to claim 1 wherein
R₁ is hydrogen, -C₁-C₆ alkoxy, -C₁-C₆ alkylthio, halogen or -NO₂,
R₂ is hydrogen or -C₁-C₆ alkoxy,
R₃ is NO₂.
R₄ is hydrogen, -C₁-C₆ alkoxy or halogen;
R₅ is hydrogen, halogen, -C₁-C₆ alkoxy, -COOH;
R₆ is hydrogen.
R₇ is hydrogen.
Rₐ is methyl or ethyl.
R_{b} is methyl or ethyl,
Y is -CH₂-COO-R₈ or -CH₂-CO-N(R₁₀)-R₉; or -CH₂-CO-N(R₁₀)-L-N(R₁₀) CO-CH₂- or Y is -CH₂-CO-O-L'-O-CO-CH₂- wherein
R₈ is C₃-C₈alkyl.
R₉ is phenyl, mesityl, phenyl-O-phenyl, phenyl-S-phenyl, phenyl once or more than once substituted by halogen, -CF₃, C₁-C₆alkyl, -C₁-C₆ alkoxy, carboxy, -COO-C₁-C₆alkyl.
R₁₀ is hydrogen, C₁-C₆alkyl, more preferably hydrogen.
L and L' independently of one another are 1,3 phenylene or 1,4 phenylene wherein the phenylene linker is optionally substituted once or more than once by halogen, -CF₃, -C₁-C₆ alkyl, -C₁-C₆ alkoxy, carboxy , -COO-C₁-C₆alkyl, -CONH₂, -CON(C₁-C₆alkyl)₂, nitro; or L is naphthalene, biphenylene or phenylene-O-phenylene.

3. A time temperature indicator according to claim 1 wherein
R₁ is hydrogen or methoxy or methylthio;
R₂ is hydrogen or methoxy;
R₃ is nitro;
R₄ is hydrogen or methoxy;
R₅ is hydrogen, halogen, methoxy or -COOH;
Rₐ is methyl or ethyl;
R_{b} is methyl or ethyl;
Y is -CH₂-COO-R₈ or -CH₂-CO-N(R₁₀)-R₉; or -CH₂-CO-N(R₁₀)-L-N(R₁₀) CO-CH₂- or Y is -CH₂-CO-O-L'-O-CO-CH₂- wherein
R₈ is C₃-C₆alkyl;
R₉ is phenyl, mesityl, phenyl-O-phenyl, phenyl-S-phenyl ,phenyl once or more than once substituted by halogen, CF₃, C₁-C₆alkyl, -C₁-C₆ alkoxy, carboxy, -COO-C₁-C₆alkyl;
R₁₀ is hydrogen;
L and L' independently of one another are 1,3 phenylene or 1,4 phenylene wherein the phenylene linker is optionally substituted once or more than once by halogen, -CF₃, C₁-C₆alkyl, -C₁-C₆ alkoxy, carboxy , -COO-C₁-C₆alkyl, -CONH₂, -CON(C₁-C₆alkyl)₂, nitro; or L is naphthalene, biphenylene or phenylene-O-phenylene.

4. A time temperature indicator according to any one of claims 1-3,
wherein Y is CH₂-CO-N(R₁₀)-R₉.

5. A time temperature indicator according to any one of claims 1-3,
wherein Y is -CH₂-COO-R₈.

6. A time temperature indicator according to any one of claims 1-3,
wherein Y is -CH₂-CO-N(R₁₀)-L-N(R₁₀) CO-CH₂-.

7. A time temperature indicator according to any one of claims 1-3,wherein Y is -CH₂-COO-L'-O-CO-CH₂

8. The use of spiropyrans of the formula I' for manufacturing a time temperature indicator 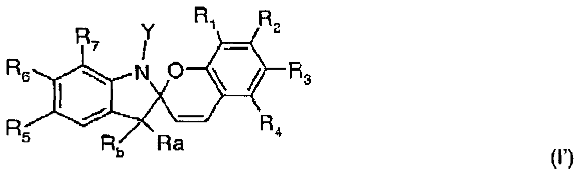 wherein
R₁ is hydrogen, -C₁-C₁₈ alkoxy, -C₁-C₁₈ alkylthio, -C₁-C₁₈ alkyl-SO-, -C₁-C₁₈ alkyl-SO₂-, phenylthio, phenyl, halogen, -C₁-C₁₈ alkyl or -NO₂;
R₂ is hydrogen or -C₁-C₁₈ alkoxy;
R₃ is NO₂ or halogen;
R₄ is hydrogen, -C₁-C₁₈ alkoxy or halogen;
R₅ is hydrogen, halogen, -C₁-C₁₈ alkoxy, -COOH, -COO-C₁-C₁₈alkyl, -CF₃ or phenyl;
R₆ is hydrogen or R₆ and R₇ form together a phenyl ring;
R₇ is hydrogen;
Rₐ is hydrogen or -C₁-C₆ alkyl;
R_{b} is hydrogen or -C₁-C₆ alkyl, or together with Rₐ form a 5-6 membered ring;
Y is -CH₂-COO-R₈ or -CH₂-CO-N(R₁₀)-R₉; or -CH₂-CO-N(R₁₀)-L-N(R₁₀) CO-CH₂- or Y is -CH₂-CO-O-L'O-CO-CH₂-
wherein
R₈ is hydrogen, C₁-C₁₈alkyl;
R₉ is phenyl, mesityl, or a higher than naphthyl annelated aromatic system, phenyl-O-phenyl, phenyl-S-phenyl, or phenyl, or thehigher than naphthyl annelated aromatic system is once or more than once substituted by halogen, -CF₃, C₁-C₆alkyl, -C₁-C₆ alkoxy, carboxy, -COO-C₁-C₆alkyl. CN, NO₂, N(R₁₁)₂, S-R₁₁(SO-R₁₁, SO₂-R₁₁), CO-R₁₁, CO-N(R₁₁)₂ (R11=C1-C18alkyl, aryl, substituted aryl) whereby in case of a more than once substitution, the substituent can be the same or different;
R₁₀ is hydrogen, C₁-C₁₈alkyl;
L is 1,3 phenylene or 1,4 phenylene wherein the phenylene linker is optionally substituted by once or more than once by halogen, -CF₃, C₁-C₁₈alkyl, -C₁-C₁₈ alkoxy, carboxy , -COO-C₁-C₁₈alkyl, -CONH₂, -CON(C₁-C₁₈alkyl)₂, nitro; or L is naphthalene, biphenylene or phenylene-O-phenylene wherein the naphthalene, biphenylene or phenylene-O-phenylene linker is optionally substituted once or more than once by halogen, -CF₃, C₁-C₁₈alkyl, -C₁-C₁₈ alkoxy, carboxy , -COO-C₁-C₁₈alkyl, -CONH₂,-CON(C₁-C₁₈alkyl)₂, nitro.
L' is 1,3 phenylene or 1,4 phenylene wherein the phenylene linker is optionally substituted by once or more than once by halogen, -CF₃, C₁-C₁₈alkyl, -C₁-C₁₈ alkoxy, carboxy , -COO-C₁-C₁₈alkyl, -CONH₂, -CON(C₁-C₁₈alkyl)₂, nitro; or L is naphthalene, biphenylene or phenylene-O-phenylene wherein the naphthalene, biphenylene or phenylene-O-phenylene linker is optionally substituted once or more than once by halogen, -CF₃, C₁-C₁₈alkyl, -C₁-C₁₈ alkoxy, carboxy , -COO-C₁-C₁₈alkyl, -CONH₂,-CON(C₁-C₁₈alkyl)₂, nitro.

9. A method of manufacturing a time-temperature indicator comprising at least one of the spiroaromatic indicator compounds of the formula I; said method comprising the steps of
(a) introducing into a matrix or atop a matrix a spiropyran indicator of the formula I as defined in claim 1 or in claim 8 and
(b) converting the spiropyran indicator from an original stable state into a metastable state by a process selected from photonic induction, thermal induction, pressure induction, electrical induction, or chemical induction,
(c) optionally applying a protector film

10. A method of determining the time temperature history of perishable goods, which method comprises the following steps:
a) printing onto a substrate a time-temperature integrator which comprises at least one spiroaromatic indicator compound as defined in claim 1 or in claim 8;
b) activating the indicator, preferably by photo-induced coloration
c) optionally applying a protector that prevents renewed photo-induced coloration of the indicator, and
d) determining the degree of time- or temperature-induced decoloration and, taking account of the degree of decoloration, the quality of the product.

11. A printing ink or printing ink concentrate, comprising at least one spiropyran indicator of the formula (I) as defined in claim 1 or in claim 8; for manufacturing a time temperature indicator.

## Patentansprüche

1. Zeit-Temperatur-Indikator, umfassend mindestens einen Spiropyran-Indikator der Formel (I) 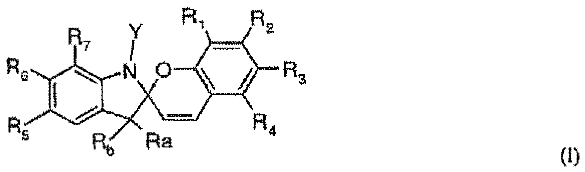 worin
R₁ Wasserstoff, -C₁-C₁₈-Alkoxy, -C₁-C₁₈-Alkylthio, -C₁-C₁₈-Alkyl-SO-, -C₁-C₁₈-Alkyl-SO₂-, Phenylthio, Phenyl, Halogen, -C₁-C₁₈-Alkyl oder -NO₂ ist;
R₂ Wasserstoff oder -C₁-C₁₈-Alkoxy ist;
R₃ NO₂ oder Halogen ist;
R₄ Wasserstoff, -C₁-C₁₈-Alkoxy oder Halogen ist;
R₅ Wasserstoff, Halogen, -C₁-C₁₈-Alkoxy, -COOH, -COO-C₁-C₁₈-Alkyl, -CF₃ oder Phenyl ist;
R₆ Wasserstoff ist oder R₆ und R₇ zusammen einen Phenylring bilden;
R₇ Wasserstoff ist;
Rₐ Wasserstoff oder -C₁-C₆-Alkyl ist;
R_{b} Wasserstoff oder -C₁-C₆-Alkyl ist oder zusammen mit Rₐ einen 5-6-gliedrigen Ring bildet;
Y -CH₂-COO-R₈ oder -CH₂-CO-N(R₁₀)-R₉; oder -CH₂-CO-N(R₁₀)-L-N(R₁₀)CO-CH₂- ist; oder Y -CH₂-CO-O-L'-O-CO-CH₂- ist, worin
R₈ Wasserstoff, C₃-C₁₈-Alkyl ist oder R₈ Ethyl ist, mit der Maßgabe, dass R₆ und R₇ zusammen einen Phenyl-ring bilden;
R₉ Phenyl, Mesityl oder ein höheres als ein Naphthyl-verschmolzenes aromatisches System, Phenyl-O-Phenyl, Phenyl-S-Phenyl ist, oder Phenyl oder das höhere als ein Naphthyl-verschmolzenes aromatisches System einmal oder mehr als einmal durch Halogen, -CF₃, C₁-C₆-Alkyl, -C₁-C₆-Alkoxy, Carboxy,-COO-C₁-C₆-alkyl, CN, NO₂, N(R₁₁)₂, S-R₁₁ (SO-R₁₁, SO₂-R₁₁), CO-R₁₁, CO-N(R₁₁)₂ (R₁₁=C₁-C₁₈-Alkyl, Aryl, substituiertes Aryl) substituiert ist, wobei im Fall einer mehr als einmaligen Substitution der Substi-tuent identisch oder verschieden sein kann;
R₁₀ Wasserstoff, C₁-C₁₈-Alkyl ist;
L 1,3-Phenylen oder 1,4-Phenylen ist, wobei der Phenylen-Linker gegebenenfalls einmal oder mehr als einmal durch Halogen, -CF₃, C₁-C₁₈-Alkyl, -C₁-C₁₈-Alkoxy, Carboxy, -COO-C₁-C₁₈-alkyl, CONH₂, -CON(C₁-C₁₈-alkyl)₂, Nitro substituiert ist; oder L Naphthalen, Biphenylen oder Phenylen-O-Phenylen ist, wobei der Naphthalen-, Biphenylen- oder Phenylen-O-Phenylen-Linker gegebenenfalls einmal oder mehr als einmal durch Halogen, -CF₃, C₁-C₁₈-Alkyl, -C₁-C₁₈-Alkoxy, Carboxy, -COO-C₁-C₁₈-alkyl, CONH₂,-CON (C₁-C₁₈-alkyl)₂, Nitro substituiert ist;
L' 1,3-Phenylen oder 1,4-Phenylen ist, wobei der Phenylen-Linker gegebenenfalls einmal oder mehr als einmal durch Halogen, -CF₃, C₁-C₁₈-Alkyl, -C₁-C₁₈-Alkoxy, Carboxy, -COO-C₁-C₁₈-alkyl, -CONH₂, -CON (C₁- C₁₈-alkyl)₂, Nitro substituiert ist; oder L Naphthalen, Biphenylen oder Phenylen-O-Phenylen ist, wobei der Naphthalen-, Biphenylen- oder Phenylen-O-Phenylen-Linker gegebenenfalls einmal oder mehr als einmal durch Halogen, -CF₃, C₁-C₁₈-Alkyl, -C₁-C₁₈-Alkoxy, Carboxy, -COO-C₁-C₁₈-Alkyl, -CONH₂,-CON (C₁-C₁₈-alkyl) ₂, Nitro substituiert ist.

2. Zeit-Temperatur-Indikator gemäß Anspruch 1, wobei
R₁ Wasserstoff, -C₁-C₆-Alkoxy, -C₁-C₆-Alkylthio, Halogen oder -NO₂ ist;
R₂ Wasserstoff oder -C₁-C₆-Alkoxy ist;
R₃ NO₂ ist;
R₄ Wasserstoff, -C₁-C₆-Alkoxy oder Halogen ist;
R₅ Wasserstoff, Halogen, -C₁-C₆-Alkoxy, -COOH ist;
R₆ Wasserstoff ist;
R₇ Wasserstoff ist;
Rₐ Methyl oder Ethyl ist;
R_{b} Methyl oder Ethyl ist;
Y -CH₂-COO-R₈ oder -CH₂-CO-N(R₁₀)-R₉; oder -CH₂-CO-N(R₁₀)-L-N(R₁₀)CO-CH₂- ist; oder
Y -CH₂-CO-O-L'-O-CO-CH₂- ist, worin
R₈ C₃-C₆-Alkyl ist.
R₉ Phenyl, Mesityl, Phenyl-O-Phenyl, Phenyl-S-Phenyl, Phenyl, einmal oder mehr als einmal durch Halogen, -CF₃, C₁-C₆-Alkyl, -C₁-C₆-Alkoxy, Carboxy, -COO-C₁-C₆-Alkyl substituiert, ist;
R₁₀ Wasserstoff, C₁-C₆-Alkyl oder stärker bevorzugt Wasserstoff ist;
L und L' unabhängig voneinander 1,3-Phenylen oder 1,4-Phenylen sind, wobei der Phenylen-Linker gegebenenfalls einmal oder mehr als einmal durch Halogen, -CF₃, C₁-C₆-Alkyl, -C₁-C₆-Alkoxy, Carboxy, -COO-C₁-C₆-alkyl, -CONH₂, -CON(C₁-C₆-alkyl) ₂, Nitro substituiert ist; oder L Naphthalen, Biphenylen oder Phenylen-O-Phenylen ist.

3. Zeit-Temperatur-Indikator gemäß Anspruch 1, worin
R₁ Wasserstoff oder Methoxy oder Methylthio ist;
R₂ Wasserstoff oder Methoxy ist;
R₃ Nitro ist;
R₄ Wasserstoff oder Methoxy ist;
R₅ Wasserstoff, Halogen, Methoxy oder -COOH ist;
Rₐ Methyl oder Ethyl ist;
R_{b} Methyl oder Ethyl ist;
Y -CH₂-COO-R₈ oder -CH₂-CO-N(R₁₀)-R₉; oder -CH₂-CO-N (R₁₀) -L-N (R₁₀)CO-CH₂- ist; oder Y -CH₂-CO-O-L'-O-CO-CH₂- ist, worin
R₈ C₃-C₆-Alkyl ist;
R₉ Phenyl, Mesityl, Phenyl-O-Phenyl, Phenyl-S-Phenyl, Phenyl, einmal oder mehr als einmal durch Halogen, -CF₃, C₁-C₆-Alkyl, C₁-C₆-Alkoxy, Carboxy, -COO-C₁-C₆-alkyl substituiert, ist;
R₁₀ Wasserstoff ist;
L und L' unabhängig voneinander 1,3-Phenylen oder 1,4-Phenylen sind, wobei der Phenylen-Linker gegebenenfalls einmal oder mehr als einmal durch Halogen, -CF₃, C₁-C₆-Alkyl, -C₁-C₆-Alkoxy, Carboxy,-COO-C₁-C₆-alkyl, -CONH₂, -CON(C₁-C₆-alkyl)₂, Nitro substituiert ist; oder L Naphthalen, Biphenylen oder Phenylen-O-Phenylen ist.

4. Zeit-Temperatur-Indikator gemäß einem der Ansprüche 1 - 3,
wobei Y -CH₂-CO-N(R₁₀)-R₉ ist.

5. Zeit-Temperatur-Indikator gemäß einem der Ansprüche 1 - 3,
wobei Y -CH₂-COO-R₈ ist.

6. Zeit-Temperatur-Indikator gemäß einem der Ansprüche 1 - 3,
wobei Y -CH₂-CO-N(R₁₀)-L-N(R₁₀)CO-CH₂- ist.

7. Zeit-Temperatur-Indikator gemäß einem der Ansprüche 1 - 3,
wobei Y -CH₂-CO-O-L'-O-CO-CH₂ ist.

8. Verwendung von Spiropyranen der Formel (I') zur Herstellung eines Zeit-Temperatur-Indikators 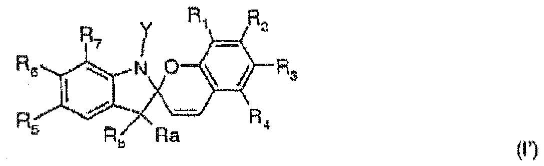 worin
R₁ Wasserstoff, -C₁-C₁₈-Alkoxy, -C₁-C₁₈-Alkylthio, -C₁-C₁₈-Alkyl-SO-, -C₁-C₁₈-Alkyl-SO₂-, Phenylthio, Phenyl, Halogen, -C₁-C₁₈-Alkyl oder -NO₂ ist;
R₂ Wasserstoff oder -C₁-C₁₈-Alkoxy ist;
R₃ NO₂ oder Halogen ist;
R₄ Wasserstoff, -C₁-C₁₈-Alkoxy oder Halogen ist;
R₅ Wasserstoff, Halogen, -C₁-C₁₈-Alkoxy, -COOH, -COO-C₁-C₁₈-Alkyl, -CF₃ oder Phenyl ist;
R₆ Wasserstoff ist oder R₆ und R₇ zusammen einen Phenylring bilden;
R₇ Wasserstoff ist;
Rₐ Wasserstoff oder -C₁-C₆-Alkyl ist;
R_{b} Wasserstoff oder -C₁-C₆-Alkyl ist oder zusammen mit Rₐ einen 5-6-gliedrigen Ring bildet;
Y -CH₂-COO-R₈ oder -CH₂-CO-N (R₁₀) -R₉; oder -CH₂-CO-N(R₁₀)-L-N(R₁₀)CO-CH₂- ist; oder Y -CH₂-CO-O-L'-O-CO-CH₂- ist, worin
R₈ Wasserstoff, C₁-C₁₈-Alkyl ist;
R₉ Phenyl, Mesityl oder ein höheres als ein Naphthylverschmolzenes aromatisches System, Phenyl-O-Phenyl, Phenyl-S-Phenyl ist, oder Phenyl oder das höhere als ein Naphthyl-verschmolzenes aromatisches System einmal oder mehr als einmal durch Halogen, -CF₃, C₁-C₆-Alkyl, -C₁-C₆-Alkoxy, Carboxy,-COO-C₁-C₆-alkyl, CN, NO₂, N(R₁₁)₂, S-R₁₁ (SO-R₁₁, SO₂-R₁₁), CO-R₁₁, CO-N(R₁₁)₂ (R₁₁=C₁-C₁₈-Alkyl, Aryl, substituiertes Aryl) substituiert ist, wobei im Fall einer mehr als einmaligen Substitution der Substituent identisch oder verschieden sein kann;
R₁₀ Wasserstoff, C₁-C₁₈-Alkyl ist;
L 1,3-Phenylen oder 1,4-Phenylen ist, wobei der Phenylen-Linker gegebenenfalls einmal oder mehr als einmal durch Halogen, -CF₃, C₁-C₁₈-Alkyl, -C₁-C₁₈-Alkoxy, Carboxy, -COO-C₁-C₁₈-alkyl, CONH₂, -CON(C₁-C₁₈-alkyl)₂, Nitro substituiert ist; oder L Naphthalen, Biphenylen oder Phenylen-O-Phenylen ist, wobei der Naphthalen-, Biphenylen- oder Phenylen-O-Phenylen-Linker gegebenenfalls einmal oder mehr als einmal durch Halogen, -CF₃, C₁-C₁₈-Alkyl, -C₁-C₁₈-Alkoxy, Carboxy, -COO-C₁-C₁₈-alkyl, CONH₂,-CON(C₁-C₁₈-alkyl)₂, Nitro substituiert ist;
L' 1,3-Phenylen oder 1,4-Phenylen ist, wobei der Phenylen-Linker gegebenenfalls einmal oder mehr als einmal durch Halogen, -CF₃, C₁-C₁₈-Alkyl, -C₁-C₁₈-Alkoxy, Carboxy, -COO-C₁-C₁₈-alkyl, -CONH₂, -CON(C₁-C₁₈-alkyl)₂, Nitro substituiert ist; oder L Naphthalen, Biphenylen oder Phenylen-O-Phenylen ist, wobei der Naphthalen-, Biphenylen- oder Phenylen-O-Phenylen-Linker gegebenenfalls einmal oder mehr als einmal durch Halogen, -CF₃, C₁-C₁₈-Alkyl, -C₁-C₁₈-Alkoxy, Carboxy, -COO-C₁-C₁₈-Alkyl, -CONH₂,-CON(C₁-C₁₈-alkyl)₂, Nitro substituiert ist.

9. Verfahren zur Herstellung eines Zeit-Temperatur-Indikators, umfassend mindestens eine der spiroaromatischen Indikatorverbindungen der Formel I; wobei das Verfahren die folgenden Schritte umfasst:
(a) Einführen in eine Matrix oder auf die Oberseite einer Matrix eines Spiropyran-Indikators der Formel I, wie in Anspruch 1 oder in Anspruch 8 definiert; und
(b) Umwandeln des Spiropyran-Indikators von einem ursprünglichen stabilen Zustand in einen metastabilen Zustand durch einen Prozess, gewählt aus photonischer Induktion, thermischer Induktion, Druckinduktion, elektrischer Induktion oder chemischer Induktion;
(c) gegebenenfalls Aufbringen eines Schutzfilms.

10. Verfahren zur Bestimmung des Zeit-TemperaturVerlaufs von verderblichen Waren, wobei das Verfahren die folgenden Schritte umfasst:
a) Drucken auf ein Substrat eines Zeit-Temperatur-Integrators, welcher mindestens eine spiroaromatische Indikatorverbindung wie in Anspruch 1 oder in Anspruch 8 definiert umfasst;
b) Aktivieren des Indikators, vorzugsweise durch photoinduzierte Verfärbung;
c) gegebenenfalls Aufbringen eines Protektors, welcher eine erneuerte photoinduzierte Verfärbung des Indikators verhindert; und
d) Bestimmen des Grades der Zeit- oder Temperaturinduzierten Entfärbung und, unter Berücksichtigung des Grades der Entfärbung, der Qualität des Produkts.

11. Drucktinte oder Drucktintenkonzentrat, umfassend mindestens einen Spiropyran-Indikator der Formel (I) wie in Anspruch 1 oder in Anspruch 8 definiert; für die Herstellung eines Zeit-Temperatur-Indikators.

## Revendications

1. Indicateur temps-température comprenant au moins un indicateur à base de spiropyrane de formule (I) 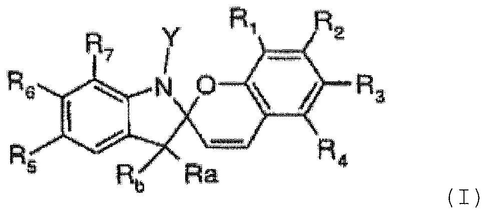 dans laquelle
R₁ représente un hydrogène, un groupe alcoxy en C₁-C₁₈, (alkyl en C₁-C₁₈)thio, (alkyl en C₁-C₁₈)-SO-, (alkyl en C₁-C₁₈)-SO₂-, phénylthio, phényle, halogéno, alkyle en C₁-C₁₈ ou -NO₂ ;
R₂ représente un hydrogène ou un groupe alcoxy en C₁-C₁₈ ;
R₃ représente NO₂ ou un halogène ;
R₄ représente un hydrogène, un groupe alcoxy en C₁-C₁₈ ou un halogène ;
R₅ représente un hydrogène, un halogène, un groupe alcoxy en C₁-C₁₈, -COOH, -COO-alkyle en C₁-C₁₈, -CF₃ ou phényle ;
R₆ représente un hydrogène, ou bien R₆ et R₇ forment conjointement un noyau phényle ;
R₇ représente un hydrogène ;
Rₐ représente un hydrogène ou un groupe alkyle en C₁-C₆ ;
R_{b} représente un hydrogène ou un groupe alkyle en C₁-C₆, ou conjointement avec Rₐ, forme un cycle à 5-6 chaînons ; et
Y représente -CH₂-COO-R₈ ou -CH₂-CO-N(R₁₀)-R₉ ; ou -CH₂-CO-N(R₁₀)-L-N(R₁₀)-CO-CH₂- ou -CH₂-CO-O-L'-O-CO-CH₂-
dans lesquels
R₈ représente un hydrogène ou un groupe alkyle en C₃-C₁₈, ou bien R₈ représente un groupe éthyle à condition que R₆ et R₇ forment conjointement un noyau phényle ;
R₉ représente un phényle, un mésityle ou un système aromatique cyclique supérieur au naphtyle, un groupe phényl-O-phényle ou phényl-S-phényle, ou bien le phényle ou le système aromatique cyclique supérieur au naphtyle est substitué une ou plusieurs fois par un halogène, un groupe -CF₃, alkyle en C₁-C₆, alcoxy en C₁-C₆, carboxyle, -COO-alkyle en C₁-C₆, CN, NO₂, N(R₁₁)₂, S-R₁₁ (SO-R₁₁, SO₂-R₁₁), CO-R₁₁, CO-N(R₁₁)₂ (R₁₁ = groupe alkyle, aryle ou aryle substitué en C₁-C₁₈), d' où il résulte qu'en cas de plusieurs substitutions, les substituants peuvent être identiques ou différents ; R₁₀ représente un hydrogène ou un groupe alkyle en C₁-C₁₈ ;
L représente un groupe 1,3-phénylène ou 1,4-phénylène, le groupe de liaison phénylène étant éventuellement substitué une ou plusieurs fois par un halogène, un groupe -CF₃, alkyle en C₁-C₁₈, alcoxy en C₁-C₁₈, carboxyle, -COO-alkyle en C₁-C₁₈, -CONH₂, -CON (alkyle en C₁-C₁₈)₂ ou nitro ; ou bien L représente un groupe naphtalène, biphénylène ou phénylène-O-phénylène, le groupe de liaison naphtalène, biphénylène ou phénylène-O-phénylène étant éventuellement substitué une ou plusieurs fois par un halogène, un groupe -CF₃, alkyle en C₁-C₁₈, alcoxy en C₁-C₁₈, carboxyle, -COO-alkyle en C₁-C₁₈, -CONH₂, -CON(alkyle en C₁-C₁₈)₂ ou nitro ; et
L' représente un groupe 1,3-phénylène ou 1,4-phénylène, le groupe de liaison phénylène étant éventuellement substitué une ou plusieurs fois par un halogène, un groupe -CF₃, alkyle en C₁-C₁₈, alcoxy en C₁-C₁₈, carboxyle, -COO-alkyle en C₁-C₁₈, -CONH₂, -CON(alkyle en C₁-C₁₈)₂ ou nitro ; ou bien L représente un groupe naphtalène, biphénylène ou phénylène-O-phénylène, le groupe de liaison naphtalène, biphénylène ou phénylène-O-phénylène étant éventuellement substitué une ou plusieurs fois par un halogène, un groupe -CF₃, alkyle en C₁-C₁₈, alcoxy en C₁-C₁₈, carboxyle, -COO-alkyle en C₁-C₁₈, -CONH₂, -CON(alkyle en C₁-C₁₈)₂ ou nitro.

2. Indicateur temps-température selon la revendication 1 dans lequel
R₁ représente un hydrogène, un groupe alcoxy en C₁-C₆, (alkyl en C₁-C₆) thio, halogéno ou -NO₂ ;
R₂ représente un hydrogène ou un groupe alcoxy en C₁-C₆ ;
R₃ représente NO₂ ;
R₄ représente un hydrogène, un groupe alcoxy en C₁-C₆ ou un halogène ;
R₅ représente un hydrogène, un halogène, un groupe alcoxy en C₁-C₆ ou -COOH ;
R₆ représente un hydrogène ;
R₇ représente un hydrogène ;
Rₐ représente un groupe méthyle ou éthyle ;
R_{b} représente un groupe méthyle ou éthyle ; et
Y représente -CH₂-COO-R₈ ou -CH₂-CO-N(R₁₀)-R₉ ; ou -CH₂-CO-N (R₁₀)-L-N (R₁₀) -CO-CH₂- ou -CH₂-CO-O-L' -O-CO-CH₂-
dans lesquels
R₈ représente un groupe alkyle en C₃-C₆ ;
R₉ représente un groupe phényle, mésityle, phényl-O-phényle, phényl-S-phényle ou phényle substitué une ou plusieurs fois par un halogène, un groupe -CF₃, alkyle en C₁-C₆, alcoxy en C₁-C₆, carboxyle ou -COO-alkyle en C₁-C₆ ;
R₁₀ représente un hydrogène ou un groupe alkyle en C₁-C₆, de préférence un hydrogène ; et
L et L', indépendamment l'un de l'autre, représentent un groupe 1,3-phénylène ou 1,4-phénylène, le groupe de liaison phénylène étant éventuellement substitué une ou plusieurs fois par un halogène, un groupe -CF₃, alkyle en C₁-C₆, alcoxy en C₁-C₆, carboxyle, -COO-alkyle en C₁-C₆, -CONH₂, -CON (alkyle en C₁-C₆)₂ ou nitro ; ou bien L représente un groupe naphtalène, biphénylène ou phénylène-O-phénylène.

3. Indicateur temps-température selon la revendication 1 dans lequel
R₁ représente un hydrogène ou un groupe méthoxy ou méthylthio ;
R₂ représente un hydrogène ou un groupe méthoxy ;
R₃ représente un groupe nitro ;
R₄ représente un hydrogène ou un groupe méthoxy ;
R₅ représente un hydrogène, un halogène, un groupe méthoxy ou -COOH ;
Rₐ représente un groupe méthyle ou éthyle ;
R_{b} représente un groupe méthyle ou éthyle ; et
Y représente -CH₂-COO-R₈ ou -CH₂-CO-N(R₁₀)-R₉ ; ou -CH₂-CO-N (R₁₀) -L-N (R₁₀)-CO-CH₂- ou -CH₂-CO-O-L' -O-CO-CH₂-
dans lesquels
R₈ représente un groupe alkyle en C₃-C₆ ; R₉ représente un groupe phényle, mésityle, phényl-O- phényle, phényl-S-phényle ou phényle substitué une ou plusieurs fois par un halogène, un groupe -CF₃, alkyle en C₁-C₆, alcoxy en C₁-C₆, carboxyle ou -COO-alkyle en C₁-C₆ ;
R₁₀ représente un hydrogène ; et
L et L', indépendamment l'un de l'autre, représentent un groupe 1,3-phénylène ou 1,4-phénylène, le groupe de liaison phénylène étant éventuellement substitué une ou plusieurs fois par un halogène,
un groupe -CF₃, alkyle en C₁-C₆, alcoxy en C₁-C₆, carboxyle, -COO-alkyle en C₁-C₆, -CONH₂, -CON(alkyle en C₁-C₆)₂ ou nitro ; ou bien L représente un groupe naphtalène, biphénylène ou phénylène-O-phénylène.

4. Indicateur temps-température selon l'une quelconque des revendications 1 à 3, dans lequel Y représente -CH₂-CO-N(R₁₀)-R₉.

5. Indicateur temps-température selon l'une quelconque des revendications 1 à 3, dans lequel Y représente -CH₂-COO-R₈.

6. Indicateur temps-température selon l'une quelconque des revendications 1 à 3, dans lequel Y représente -CH₂-CO-N (R₁₀) -L-N (R₁₀)-CO-CH₂-.

7. Indicateur temps-température selon l'une quelconque des revendications 1 à 3, dans lequel Y représente -CH₂-CO-O-L'-O-CO-CH₂-.

8. Utilisation de spiropyranes de formule (I') pour la fabrication d'un indicateur temps-température 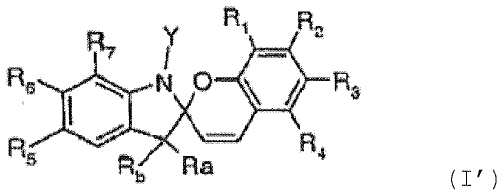 dans laquelle
R₁ représente un hydrogène, un groupe alcoxy en C₁-C₁₈, (alkyl en C₁-C₁₈)thio, (alkyl en C₁-C₁₈)-SO-, (alkyl en C₁-C₁₈)-SO₂-, phénylthio, phényle, halogéno, alkyle en C₁-C₁₈ ou -NO₂ ;
R₂ représente un hydrogène ou un groupe alcoxy en C₁-C₁₈ ;
R₃ représente NO₂ ou un halogène ;
R₄ représente un hydrogène, un groupe alcoxy en C₁-C₁₈ ou un halogène ;
R₅ représente un hydrogène, un halogène, un groupe alcoxy en C₁-C₁₈, -COOH, -COO-alkyle en C₁-C₁₈, -CF₃ ou phényle ;
R₆ représente un hydrogène, ou bien R₆ et R₇ forment conjointement un noyau phényle ;
R₇ représente un hydrogène
Rₐ représente un hydrogène ou un groupe alkyle en C₁-C₆ ;
R_{b} représente un hydrogène ou un groupe alkyle en C₁-C₆, ou conjointement avec Rₐ, forme un cycle à 5-6 chaînons ; et
Y représente -CH₂-COO-R₈ ou -CH₂-CO-N(R₁₀)-R₉ ; ou -CH₂-CO-N (R₁₀) -L-N (R₁₀) -CO-CH₂- ou -CH₂-CO-O-L' -O-CO-CH₂-
dans lesquels
R₈ représente un hydrogène ou un groupe alkyle en C₁-C₁₈ ;
R₉ représente un phényle, un mésityle ou un système aromatique cyclique supérieur au naphtyle, un groupe phényl-O-phényle ou phényl-S-phényle, ou bien le phényle ou le système aromatique cyclique supérieur au naphtyle est substitué une ou plusieurs fois par un halogène, un groupe -CF₃, alkyle en C₁-C₆, alcoxy en C₁-C₆, carboxyle, -COO-alkyle en C₁-C₆, CN, NO₂, N (R₁₁)₂, S-R₁₁ (SO-R₁₁, SO₂-R₁₁), CO-R₁₁, CO-N (R₁₁)₂ (R₁₁ = groupe alkyle, aryle ou aryle substitué en C₁-C₁₈), d'où il résulte qu'en cas de plusieurs substitutions, les substituants peuvent être identiques ou différents ;
R₁₀ représente un hydrogène ou un groupe alkyle en C₁-C₁₈ ;
L représente un groupe 1,3-phénylène ou 1,4-phénylène, le groupe de liaison phénylène étant éventuellement substitué une ou plusieurs fois par un halogène, un groupe -CF₃, alkyle en C₁-C₁₈, alcoxy en C₁-C₁₈, carboxyle, -COO-alkyle en C₁-C₁₈, -CONH₂, -CON (alkyle en C₁-C₁₈)₂ ou nitro ; ou bien L représente un groupe naphtalène, biphénylène ou phénylène-O-phénylène, le groupe de liaison naphtalène, biphénylène ou phénylène-O-phénylène étant éventuellement substitué une ou plusieurs fois par un halogène, un groupe -CF₃, alkyle en C₁-C₁₈, alcoxy en C₁-C₁₈, carboxyle, -COO-alkyle en C₁-C₁₈, -CONH₂, -CON(alkyle en C₁-C₁₈) ou nitro ; et
L' représente un groupe 1,3-phénylène ou 1,4-phénylène, le groupe de liaison phénylène étant éventuellement substitué une ou plusieurs fois par un halogène, un groupe -CF₃, alkyle en C₁-C₁₈, alcoxy en C₁-C₁₈, carboxyle, -COO-alkyle en C₁-C₁₈, -CONH₂, -CON(alkyle en C₁-C₁₈)₂ ou nitro ; ou bien L représente un groupe naphtalène, biphénylène ou phénylène-O-phénylène, le groupe de liaison naphtalène, biphénylène ou phénylène-O-phénylène étant éventuellement substitué une ou plusieurs fois par un halogène, un groupe -CF₃, alkyle en C₁-C₁₈, alcoxy en C₁-C₁₈, carboxyle, -COO-alkyle en C₁-C₁₈, -CONH₂, -CON(alkyle en C₁-C₁₈)₂ ou nitro.

9. Procédé de fabrication d'un indicateur temps-température comprenant l'un au moins des composés indicateurs spiroaromatiques de formule I, ledit procédé comprenant les étapes consistant à
(a) introduire dans une matrice ou au sommet d'une matrice un indicateur à base de spiropyrane de formule I tel que défini dans la revendication 1 ou dans la revendication 8,
(b) transformer l'indicateur à base de spiropyrane d'un état initial stable en un état métastable par un procédé choisi parmi l'induction photonique, l'induction thermique, l'induction par pression, l'induction électrique et l'induction chimique, et (c) appliquer éventuellement un film protecteur.

10. Procédé pour déterminer l'historique temps-température de marchandises périssables, lequel procédé comprend les étapes suivantes :
a) imprimer sur un substrat un intégrateur temps-température qui comprend au moins un composé indicateur spiroaromatique tel que défini dans la revendication 1 ou dans la revendication 8 ;
b) activer indicateur, de préférence par coloration photoinduite ;
c) appliquer éventuellement un protecteur qui empêche le renouvellement de la coloration photoinduite de l'indicateur ; et
d) déterminer le degré de décoloration induite par le temps ou la température, et en tenant compte du degré de décoloration, la qualité du produit.

11. Encre d'impression ou concentré d'encre d'impression, comprenant au moins un indicateur à base de spiropyrane de formule (I) tel que défini dans la revendication 1 ou dans la revendication 8, pour la fabrication d'un indicateur temps-température.
